# EUROPEAN PATENT APPLICATION

(11) **EP 2 764 846 A1**
(43) Date of publication of application: **13.08.2014**
(21) Application number: 14166837.6
(22) Date of filing: 07.02.2008
(51) Int. Cl.: A61F 2/16, A61F 9/007, A61F 2/14

(54) **Injector for eye**

(30) Priority: 08.02.2007 US 900103 P; 08.02.2007 US 900105 P; 05.06.2007 US 924910 P
(62) Divisional of application: 08710924.5
(71) Applicant: Kaneka Corporation, Osaka (JP)
(72) Inventor: Ide, Takeshi, Kobe-shi Hyogo, 658-0063 (JP); Yamamoto, Kazuaki, Osaka, 566-0072 (JP); Fukaya, Kohei, Osaka, 566-0072 (JP); Nakano, Ryoji, Osaka, 566-0072 (JP)
(74) Representative: Gille Hrabal

(57) **Abstract**

An injector for an eye used for inserting an implant into an eyeball comprises an outer tube (11), and an implant holding structure (12,13) (middle and inner tube) arranged in the outer tube to be slidable with respect to the outer tube. The implant holding structure has an implant holding portion (13b) which can be contained in the outer tube and forms a space for containing the implant between the implant holding portion and the inner surface of the outer tube when it is contained in the outer tube. The implant holding portion projects from the outer tube when the implant holding structure is advanced and the implant can be taken out from the outer tube.

## Description

### Technical Field

The present invention relates to an ophthalmic medical device, and more particularly, an injector for an eye in order to deliver a graft to a transplant site and a system thereof.

### Background Art

The cornea is originally transparent, but when the corneal endothelial cell layer located a backside of the cornea becomes dysfunctional, edema or opacity sometimes occurs in the cornea to causes seriously impaired eyesight. The endothelial cell layer has the barrier function for protecting the corneal stroma from penetration of intraocular fluid (main components: collagen and proteoglycan) to some extent and the pump function for evacuating the penetrated intraocular fluid. When deterioration of the functions causes corneal hydrops, it is hard to transmit light. Specifically, a genetic factor (Fuchs' endothelial dystrophy), external factors such as cataract operation or corneal infection, the side effect by the laser therapy for glaucoma, or the long-term wearing of contact lens or the like causes the reduction of the number (density) of the endothelial cells, and in the late stages of the symptom, the cornea is sometimes filled with fluid to cause blisters, that is, bullous keratopathy is developed.

Generally, when a permanent opacity is observed in the diseases, because of no effective treatment by pharmaceutical drugs, penetrating keratoplasty is often the first-choice in the treatment. The penetrating keratoplasty is a medical transplantation in which the central part of an opacified cornea is cut out and a transparent cornea having the same size obtained from a donated eye (a cadaveric eye) is sutured. However, the operative procedure for treating the dysfunction of the corneal endothelium, in which all layers of the cornea (the epithelial layer, the Bowman's membrane, the corneal stroma, the Descemet's membrane, and the endothelial layer) are cut out to be replaced with a donor graft, has many problems (Non-patent Documents 1 and 2).

Under the situation, recently, an operative procedure of posterior lamellar keratoplasty (PLK) in which not all the layers but a graft mainly containing endothelial cells is replaced has been developed and the clinical studies are ongoing mainly in the United States. Technically, PLK can be classified into three groups: deep lamellar endothelial keratoplasty (DLEK) in which a donor graft is inserted through a corneoscleral incision to transplant, small incision DLEK in which a folded donor graft is inserted through a smaller incision than in DLEK to transplant, and Descemet's-stripping endothelial keratoplasty (DSEK) having a feature of Descemet's membrane stripping or Descemet's-stripping automated endothelial keratoplasty (DSAEK) using a microkeratome in DSEK, which have been developed (Non-patent Documents 3 and 4). Furthermore, the method of transplantation of only the Descemet's membrane (Non-patent Document 2) and the studies by using not the graft prepared from a donated eye but a cultured corneal endothelial cell sheet or a tissue-cultured graft have been reported (Non-patent Document 5).

However, the above mentioned operative procedures still have many problems, for example, sometimes, the endothelial graft is peeled off after transplantation and the density of the corneal endothelial cells are reduced after the operation to become retransplantation (Non-patent Document 2). These cases have the possibility that an operator strongly gripped the endothelial cell graft with tweezers and the like when insertion through a corneoscleral incision, so that the endothelial cells are damaged to affect the prognosis (Non-patent Document 6). The endothelial cells have no proliferative activity in vivo, thus once the cells lead to death, the cell density is not recovered to the original level in the lifetime, so that the handling must be performed quite carefully.

Furthermore, when a graft is inserted into an eyeball (the anterior chamber), the corneoscleral incision must be enlarged with tweezers and the like, but at this time, sometimes intraocular fluid leaks out and the anterior chamber becomes shallow (an intraocular pressure becomes low). Since such state is not preferable with respect to the ocular tissue, an operator must pay great attention so as to keep the anterior chamber (so as to keep an intraocular pressure). Considering the troubles, the medical devices are disclosed for easy operation of Deep lamellar endothelial keratoplasty (DLEK) (Patent Document 1 and Non-patent Document 7). However, even when these devices are used, a graft such as endothelial cells must be set in a medical device by using a grip tool and the like with the graft rolled, so that the risk of graft damage is still high. Furthermore, since a graft is discharged into an eyeball with the graft rolled, the possibility that the graft is not thoroughly spread from the rolled state to be abnormally arranged to a transplant site is high.
Patent Document 1: U.S. Patent Application Publication No. 2007/0208422 specification.
Non-patent Document 1: Marianne O. Price, Francis W. Price, Ophthalmology, vol. 113:11, p 1936-1942 (2006).
Non-patent Document 2: S Shimmura, H Miyashita, K Konomi, N Shinozaki, T Taguchi, H Kobayashi, J Shimazaki, J Tanaka, K Tsubota, "Transplantation of corneal endothelium with Descemet's membrane using a hydroxyethyl methacrylate polymer as a carrier", British journal of ophthalmology, vol. 89, p 134-137 (2005).
Non-patent Document 3: Gerrit R.J. Melles, Cornea, vol. 25:8, p 879-881 (2006).
Non-patent Document 4: Francis W. Price, Marianne O. Price, Journal of cataract and refractive surgery, vol: 32, p 411-418 (2006).
Non-patent Document 5: Ide T, Nishida K, Yamato M, Sumide T, Utsumi M, Nozaki T, Kikuchi A, Okano T, Tano Y, Biomaterials, vol. 24:4, p 607-614 (2006).
Non-patent Document 6: Walter Bethke, Review of ophthalmology, vol. 13:09 (2006).
Non-patent Document 7: Gerrit R.J. Melles et al., "Transplantation of Descemet's membrane carrying viable endothelium through a small scleral incision", Cornea, vol. 21:4, p 415-418 (2002).

### Disclosure of the Invention

### Technical Problems to be Solved

Considering the above problems, the present invention is to provide an injector for an eye in which a graft containing endothelial cells and the like can be inserted into an eyeball through a small incision with protection from any damage and can be easily delivered to a transplant site, and a system thereof.

### Means to Solve the Problems

The present inventors have intensively studied to solve the problems. As a result, in an injector for an eye including an outer cylinder and a graft holding structure arranged inside the outer cylinder to be slidable with respect to the outer cylinder, with regard to structures of the outer cylinder and the graft holding structure, considering a graft protection mechanism, the structure of the outer cylinder suited for insertion through a small corneoscleral incision, the graft holding structure slidable with respect to the outer cylinder (a middle cylinder and an inner cylinder), materials, sizes, and shapes thereof, a fluid discharge mechanism, an intraocular pressure keeping mechanism, a graft storing mechanism, surface coating, a mechanism for preventing over-insertion of each cylinder, an intraocular pressure relieving mechanism, and the like, the injector for an eye is designed, and the inventors found that, by using the injector for an eye and the system thereof, a graft containing endothelial cells can be inserted through a small incision into the intraocular without any damage and easily delivered to a transplant site, as well as an eyeball internal pressure (the anterior chamber formation) can be kept during and after use of the injector, and the invention has been accomplished.

That is, the summary of the present invention is described below:
(1) an injector for an eye for inserting a graft into an eyeball includes an outer cylinder and a graft holding structure arranged inside the outer cylinder to be slidable with respect to the outer cylinder. The graft holding structure has a graft holding part which can be stored inside the outer cylinder to form a space for storing a graft between an inner surface of the outer cylinder and the graft holding part when the graft holding part is stored in the outer cylinder, and the graft holding part protrudes from the outer cylinder when the graft holding structure is advanced and the graft can be taken out from the outer cylinder.
(2) In the injector for an eye according to (1), the graft holding structure includes a middle cylinder and an inner cylinder arranged in the middle cylinder to be slidable with respect to the middle cylinder, and the middle cylinder pushes out the graft of the graft holding part by sliding the middle cylinder with respect to the outer cylinder and/or sliding the middle cylinder with respect to the inner cylinder.
(3) In the injector for an eye according to (1) or (2), the graft holding part includes a fluid discharge hole toward an inner surface of the graft.
(4) The injector for an eye according to any of (1) to (3) further includes another discharge hole for liquid for keeping intraocular pressure or forming the anterior chamber.
(5) In the injector for an eye according to (4), the other discharge hole is formed on the graft holding structure.
(6) In the injector for an eye according to (5), the other discharge hole is formed on the inner cylinder, on the middle cylinder, or in a space between the inner cylinder and the middle cylinder.
(7) The injector for an eye according to any of (1) to (6) further includes a mechanism for relieving intraocular pressure.
(8) In the injector for an eye according to (7), the mechanism for relieving intraocular pressure has a structure in which an aperture at a leading end of the outer cylinder communicates with an aperture formed on a side face or a rear end part of the outer cylinder by sliding the graft holding structure toward the outside of an eye.
(9) In the injector for an eye according to (7) or (8), the mechanism for relieving intraocular pressure has a structure in which an aperture at a leading end of the outer cylinder or the middle cylinder communicates with the aperture formed on the side face or the rear end part of the outer cylinder by sliding the middle cylinder or the inner cylinder included in the graft holding structure toward the outside of an eye.
(10) In the injector for an eye according to (9), the mechanism for relieving intraocular pressure has a structure in which pressure outlets formed on the middle cylinder and the outer cylinder communicate with an inner space of the middle cylinder formed by sliding the inner cylinder toward the outside of an eye, and whereby the aperture at the leading end of the outer cylinder communicates with the pressure outlet formed on the outer cylinder.
(11) The injector for an eye according to any of (1) to (10) further includes an aperture for installing a graft, the aperture is on a side face of the outer cylinder and the side face is the position where the graft holding part is stored when the graft holding structure is arranged in the outer cylinder.
(12) The injector for an eye according to (11) further includes an opening and closing gate for sealing the aperture.
(13) In the injector for an eye according to any of (3) to (12), the fluid is air.
(14) In the injector for an eye according to any of (1) to (13), an inner surface of the outer cylinder and/or an outer surface of the graft holding structure has lubricious coating.
(15) In the injector for an eye according to (14), an outer surface of the inner cylinder and/or the middle cylinder included in the graft holding structure has lubricious coating.
(16) In the injector for an eye according to any of (1) to (15), the graft includes at least one selected from corneal endothelial cell, Descemet's membrane of cornea, corneal stroma, iris tissue, capsula lentis, retina, or a cultured composition.
(17) In the injector for an eye according to (16), the graft is a sheet piece used for transplantation of corneal endothelial cells.
(18) A method for inserting a graft into an eyeball by using an injector for an eye including an outer cylinder and a graft holding structure arranged inside the outer cylinder to be slidable with respect to the outer cylinder; includes the steps of inserting the outer cylinder into an eyeball with a graft stored between a graft holding part of the graft holding structure stored in the outer cylinder and an inner surface of the outer cylinder, and advancing the graft holding structure with respect to the outer cylinder to take out the graft held on the graft holding part form the outer cylinder.
(19) A method for inserting a graft into an eyeball by using an injector for an eye including an outer cylinder and a graft holding structure arranged inside the outer cylinder to be slidable with respect to the outer cylinder, the graft holding structure further having a middle cylinder and an inner cylinder arranged inside the middle cylinder to be slidable with respect to the middle cylinder, includes the steps of inserting the outer cylinder into an eyeball with the graft stored between the graft holding part of the inner cylinder stored in the outer cylinder and the inner surface of the outer cylinder, advancing the inner cylinder and the middle cylinder included in the graft holding structure with respect to the outer cylinder to arrange at at least a leading end of the inner cylinder further toward the distal end than a leading end of the outer cylinder, and advancing the middle cylinder with respect to the inner cylinder to push out the graft held on the graft holding part of the inner cylinder from the outer cylinder.
(20) The method for inserting the graft into an eyeball according to (18) or (19) further includes a step of discharging a fluid from the graft holding part toward an inner surface of the graft taken out from the outer cylinder.
(21) A method for an eye treatment by using any of the injector for an eye according to (1) to (17).
(22) A method for an eye treatment by using the method for inserting the graft into an eyeball according to any of (18) to (20).

### Effect of the Invention

By the invention according to (1), a graft can be held in the space formed between the graft holding part included in the graft holding structure capable of being stored in the outer cylinder and the inner surface of the outer cylinder, and further the graft holding part is protruded from the outer cylinder by advancing the graft holding structure, so that the graft can be taken out from the outer cylinder. Thus, the graft can be easily inserted and stored in the outer cylinder without using tweezers or other gripping tools, so that the graft can be stored and protected in the injector for an eye without any damage. Furthermore, the graft can be inserted into an eyeball with the graft protected in the outer cylinder, then the graft holding structure is advanced to protrude from the outer cylinder and the graft can be taken out from the outer cylinder, so that the graft can be easily delivered to a transplant site in an eyeball.

By the invention according to (2), since the graft holding structure further includes the middle cylinder and the inner cylinder, the middle cylinder can push out the graft of the graft holding part by sliding of the middle cylinder with respect to the outer cylinder and/or sliding of the middle cylinder with respect to the inner cylinder, so that the graft can be more easily delivered to a transplant site in an eyeball.

By the invention according to (3), since the graft holding part includes the fluid discharge hole toward an inner surface of the graft, the graft held on the graft holding part can be more simply and reliably lifted up by the fluid to be delivered to a transplant site.

By the invention according to (4) to (6), since the injector for an eye includes the other discharge hole for liquid for keeping intraocular pressure or forming the anterior chamber, when the injector for an eye is inserted into an eyeball through a corneoscleral incision, reduction of an intraocular pressure or contraction of the anterior chamber caused by flowing out of intraocular fluid from the corneoscleral incision and the like can be prevented.

By the invention according to (7) to (10), since the injector for an eye includes the mechanism for relieving pressure in an eyeball (intraocular pressure), when the injector for an eye is removed at the state of a high intraocular pressure, the mechanism can effectively prevent that the graft delivered to the affected area becomes unstable as well as the pressure decreases.

By the invention according to (11), since the aperture for installing the graft in the outer cylinder which stores the graft holding part is formed on a side face of the outer cylinder and the side face is where the graft holding part is stored, and by the invention according to (12), since the opening and closing gate for sealing the aperture is included, the graft can be directly installed from the aperture into the injector for an eye (the outer cylinder). Thus, graft damage caused by inserting from an aperture at the leading end of the outer cylinder can be effectively prevented. Furthermore, it has the advantage that the transfer of both ways of the graft in the outer cylinder is shortened by omitting the transfer of one way. In addition, when the opening and closing gate is included, the graft installed in the outer cylinder can be protected from sudden trouble.

By the invention according to (13) to (17), when the fluid discharging toward the inner surface of the graft is air, the safety is further improved, and since the inner surface of the outer cylinder and/or the outer surface of the graft holding structure or the like has surface coating for graft sliding, the slidability of the graft is improved, so that the graft installed in the injector for an eye can be easily pushed-out and sudden damage to the graft can be effectively prevented.

By the invention according to (18) to (22), since the graft can be inserted into an eyeball with the graft protected and easily delivered to a transplant site, the injector has high efficacy for posterior lamellar keratoplasty such as PLK, DLEK, DSEK, DSAEK, and DMEK.

### Brief Description of Drawings

Figure 1 is a perspective view showing an example of an ophthalmic medical device set using the injector for an eye.
Figure 2 is an exploded perspective view of the injector for an eye 1 showing a first embodiment of each component.
Figure 3 is a side view of the embodiment of the outer cylinder of the injector for an eye 1.
Figure 4 (a) is a cross-sectional view taken along line I-I of the graft storage part of the outer cylinder in Figure 3, and Figure 4(b) is a cross-sectional view taken along line II-II of the grip part of the outer cylinder in Figure 3.
Figure 5 is a perspective view of the outer cylinder shown in Figure 3.
Figure 6 is a side view of the embodiment of the middle cylinder of the injector for an eye 1.
Figure 7(a) is a cross-sectional view taken along line III-III of the discharge hole in Figure 6, and Figure 7(b) is a cross-sectional view taken along line IV-IV of the operational bar in Figure 6.
Figure 8 is a perspective view of the middle cylinder shown in Figure 6.
Figure 9 is a side view of the embodiment of the inner cylinder of the injector for an eye 1.
Figure 10 (a) is an enlarged sectional view of the leading end of the inner cylinder shown in Figure 9, and Figure 10 (b) is an enlarged sectional view of the Y-shaped connector proximal portion of the inner cylinder shown in Figure 9.
Figure 11 is a perspective view of the inner cylinder shown in Figure 9.
Figure 12 is a schematic view of the procedure showing an example of transplantation using the injector for an eye 1.
Figure 13 is a schematic view of the procedure showing the transplantation following Figure 12.
Figure 14 (a) is a partial perspective view showing another embodiment of the hinge structure, and Figure 14 (b) is an exploded perspective view showing another embodiment of the outer cylinder.
Figure 15 (a) is a partial side view showing another embodiment of the graft storage part of the outer cylinder, and Figure 15 (b) is a cross-sectional view taken along line V-V of the graft storage part.
Figure 16 is a partial side view showing still another embodiment of the graft storage part of the outer cylinder.
Figure 17 is a schematic perspective view showing the injector for an eye 1A as a second embodiment.
Figure 18 is a perspective view of each component of the injector for an eye 1A.
Figure 19 is a schematic view of the top surface of the outer cylinder 21 of the injector for an eye 1A.
Figure 20 (a) is a cross-sectional view taken along line VI-VI in Figure 19, Figure 20 (b) is a cross-sectional view taken along line VII-VII in Figure 19, Figure 20 (c) is a cross-sectional view taken along line VIII-VIII in Figure 19, and Figure 20(d) is a cross-sectional view taken along line IX-IX in Figure 19.
Figure 21 (a) is an enlarged fragmentary view of the leading end side of the outer cylinder 21, and Figure 21 (b) is a cross-sectional view taken along line X-X of Figure 21 (a).
Figure 22 is a perspective view of the outer cylinder 21.
Figure 23 is a schematic side view of the middle cylinder 22 of the injector for an eye 1A.
Figure 24 (a) is a cross-sectional view taken along line XI-XI of Figure 23, and Figure 24(b) is a cross-sectional view taken along line XII-XII of Figure 23.
Figure 25 is a perspective view of the middle cylinder 22.
Figure 26 is a schematic side view of the inner cylinder 23 of the injector for an eye 1A.
Figure 27 is an enlarged sectional view of the leading end of the inner cylinder 23.
Figure 28 is an enlarged sectional view of the rear end of the inner cylinder 23.
Figure 29 is a perspective view of the inner cylinder 23.
Figure 30 (a) is a schematic side view of the injector for an eye 1B as a third embodiment, Figure 30 (b) is a schematic side view of the injector for an eye 1B showing the state in which the graft holding part 33b of the inner cylinder 33 protrudes from the outer cylinder 31, and Figure 30 (c) is a side view showing the state in which the graft 7 is lifted up by a fluid in the injector for an eye 1B.
Figure 31 is a sectional view of the injector for an eye 1B shown in Figure 30 (b).
Figure 32 (a) is a schematic view showing the state in which the graft holding structure protrudes from the outer cylinder 41 of an injector for an eye 1C, Figure 32 (b) is a schematic view showing the state in which the graft holding structure is stored in the outer cylinder 41 of the injector for an eye 1C, and Figure 32 (c) is a schematic elevation view of the injector for an eye 1C shown in Figure 32 (b).
Figure 33 shows an example of the injector for an eye as the embodiment.
Figure 34 shows the installation of the graft to the injector for an eye (1).
Figure 35 shows the installation of the graft to the injector for an eye (2).
Figure 36 shows the installation of the graft to the injector for an eye (3).
Figure 37 shows the insertion of the injector for an eye into the anterior chamber.
Figure 38 shows the exposure of the graft into the anterior chamber. Figure 39 shows the separation and the expansion of the graft into the anterior chamber.
Figure 40 shows the result of toluidine blue-alizarin staining of the graft inserted by using the injector for an eye.
Figure 41 shows an example of the injector for an eye as the embodiment.
Figure 42 shows the installation of the graft to the injector for an eye (1).
Figure 43 shows the installation of the graft to the injector for an eye (2).
Figure 44 shows the installation of the graft to the injector for an eye (3).
Figure 45 shows the separation and the expansion of the graft into the anterior chamber.
Figure 46 shows the insertion of the graft into the anterior chamber by using ophthalmic tweezers.
Figure 47 shows the result of toluidine blue-alizarin staining of the graft inserted by using the ophthalmic tweezers.
Figure 48 shows an example of the injector for an eye as the embodiment.
Figure 49 shows the installation of the graft to the injector for an eye. Figure 50 shows an example of the injector for an eye as the embodiment.
Figure 51 shows the storage part of the injector for an eye.

### Brief Description of Numerals

1, 1A, 1B, 1C injector for eye
2, 2A, 2B Y-shaped connector
2a, 2Aa, 2Ba perfusion port
2b, 2Ab, 2Bb fluid discharge port
2c, 2Ac Y-shaped connector distal part
3 syringe
4 bottle
5 tube
6 tube
7 graft
8 transplantation intended position
8a the corneal stroma
8b the corneal endothelium and the Descemet's membrane
9 air
11, 21, 31, 41 outer cylinder
11a, 21a, 31a, 41a distal end
11b proximal end
11c, 21c, 31c, 41c inner space
111, 211 leading end
112, 212 graft storage part
112a, 212a aperture
112b, 212b gate
112c, 212c fit fixing part
112d, 212d lock (female)
112e, 212e lock (male)
112f, 212f hinge part
112g opening edge of gate 112b
112h aperture edge of fit fixing part 112c
112i stage of gate 112b
112j stage of fit fixing part 112c
1121f hinge
1123 hollow
113, 213 grip part
113a, 213a groove
113b, 213b distal end of groove 113a
113c, 213c notch structure
1131a groove
114, 214 pressure outlet
12, 22 middle cylinder
12a, 22a distal end
12b, 22b proximal end
12c, 22c inner space
121, 221 tube
121a, 221a outlet
122 packing
122a, 222a side face of middle cylinder distal end
123, 223 operational bar
123a, 223a distal end
13, 23, 33 inner cylinder
13a, 23a, 33a distal end
13b, 23b, 33b, 43b graft holding part
131, 231, 331 outer tube
132, 232 fluid discharge tube
132a, 232a, 332a, 432a discharge hole
133, 333 perfusion tube

### Best Mode for Carrying Out the Invention

Hereinafter, the present invention will be described in detail.

The injector for an eye of the present invention relates to an ophthalmic medical device which is available when, for example, a graft prepared for transplantation into an eyeball is inserted into an eyeball through a small corneoscleral incision and the graft is guided (hereinafter, sometimes referred to as "delivered") to a predetermined transplant site.

Namely, the injector for an eye according to the invention includes an outer cylinder and a graft holding structure arranged inside the outer cylinder to be slidable with respect to the outer cylinder. The graft holding structure has a graft holding part which can be stored inside the outer cylinder and forms a space capable of storing a graft between an inner surface of the outer cylinder and the graft holding part when the graft holding part is stored in the outer cylinder. The graft holding part protrudes from the outer cylinder when the graft holding structure is advanced, and the graft can be taken out from the outer cylinder.

Any kinds of graft such as a piece prepared for transplanting into an eyeball can be used for the graft, and preferable grafts are sheet-shaped grafts containing ocular tissues derived from a donor, cells or culture cells derived from a donor, and the like. More preferred grafts are sheet-shaped grafts from the corneal endothelium region (endothelial cells, the Descemet's membrane, or the corneal stroma), the iris tissue, the capsula lentis, or the retina which are prepared for transplantation. Specifically, any graft prepared for posterior lamellar keratoplasty so-called DSEK, DSAEK, PLK, DLEK, DMEK (Descemet's membrane endothelial keratoplasty), and the like may be used. The size of the graft is not specifically limited, but an example is a piece of a sheet-shaped thin film with a diameter of 7 to 9.5 mm used for posterior lamellar keratoplasty.

Furthermore, if required, the grafts may be coated or impregnated with viscoelastic materials, pharmaceutical drugs, or the like such as hyaluronic acid.

Hereinafter, embodiments of the injector for an eye according to the invention will be explained.

The injector for an eye according to the invention can include a graft protection mechanism so that a graft would not be damaged when the graft is inserted into an eye.

The graft protection in the invention includes, for example, protection in order not to give damage to cells contained in the graft or the graft in a process of inserting the graft into an eyeball through a small corneoscleral incision and delivering the graft to a transplant site. An example for the mechanism is the mechanism of holding a graft in the structure which can remove or reduce a load such as pressure, tension, and contraction to the graft when the graft is inserted into an eyeball through an incision. Especially, it is preferable that the structure is made of a harder material than the eyeball tissue and can safely hold a graft in the inner space to insert the graft into an eyeball.

Specifically, the graft protection mechanism can include an outer cylinder and a graft holding structure arranged inside the outer cylinder.

The outer cylinder has a cylinder shaped structure which becomes to contact an eyeball when the injector is inserted into the eye, for example, through a small corneoscleral incision and the structure can hold the graft and the graft holding structure inside the outer cylinder.

More specifically, the structure of the outer cylinder will be explained below.

It is preferable that the outer cylinder has a leading end for inserting into an eye and a grip part nearer to hand than the leading end. The leading end and the grip part have, for example, an approximately column-shaped structure and may have a cylinder-shaped structure with a communicated cylinder-shaped inner space.

The cylinder in the specification is not limited to the structure having a column-shaped inner space, but the inner space may be ellipsoidal, cubic, pistol bore-like, wavelike, polygonal, or conic in shape. The diameter of the inner space in the outer cylinder is not specifically limited, but if the inner space is column-shaped, preferably the diameter ϕ is in a range of 2 to 6 mm, and more preferably 2.5 to 4 mm. Even if the inner space is not column-shaped, from the viewpoint that a graft with a diameter of 7 to 11 mm can be stored without wrinkles, the inner perimeter is preferably 7 to 11 mm, more preferably 8 to 10 mm, and even more preferably 8.5 to 9.5 mm.

An outer structure of the outer cylinder is preferably column, ellipsoidal, cubic, or conic in shape for easy handling and gripping with fingers. The diameter of the grip part is not specifically limited, but preferably ϕ 3 to 50 mm, and even more preferably ϕ 6 to 20 mm. The length of the outer cylinder is not specifically limited, but for example 50 to 200 mm. The length in the axis direction of the leading end is preferably 8 mm or below, more preferably 6 mm or below, and even more preferably 5 mm or below.

The outer cylinder may include a function for storing a graft into the outer cylinder. As the function for storing a graft into the outer cylinder, an aperture may be formed on a side face of the outer cylinder so that the inner space could communicate with the outside. Furthermore, an opening and closing gate may be formed for closing the aperture. For example, the opening and closing gate is formed on the aperture to make a graft storage part, the gate is opened for storing a graft into the outer cylinder, and then the injector for an eye can insert into an eyeball with the gate closed. It is preferable that the aperture, the graft storage part, the opening and closing gate, and the like are formed on the grip part of the outer cylinder, and more preferably on a leading end of the grip part. Since the formation of the aperture on the position makes a travel distance of a graft short, the possibility of graft damage can be reduced. Furthermore, the opening and closing gate may open and close entire aperture or a part of the aperture, but preferably can close at least an inserting part into an eye.

The aperture may have the opening and closing gate to be opened and closed with respect to the outer cylinder by a hinge mechanism or the like.

The opening and closing gate of the invention may be a detachable gate. For example, a part of or all of the axis direction of the outer cylinder is divided on a face containing the axis of the injector for an eye (an arc included in the outer cylinder is halved) to make the gate, or the arc included in the outer cylinder is divided in 3 to 12 and a piece of them is cut to make the gate. The gate may further include a hinge mechanism by flexible plastic, hinges, or the like. The number of the hinge part depends on the divided number of the arc on the outer cylinder such as 2- to 12-division as described above, and as described later, in the case that the opened gate becomes a stage on which a graft is placed and especially the opening and closing gate is opened and closed in the perpendicular direction with respect to the axis direction of the injector for an eye, if a plurality of the hinge parts are formed, a curvature of the gate becomes small when the gate is opened and the length of the opened gate (the total length of arc chords) becomes long, so that liquid or air is hard to get into between the inner surface of the gate and the graft as well as visibility to the graft becomes better. Therefore, when the gate is closed, a graft can be stably stored in the cylinder. Accordingly, it is desirable that a plurality of hinge parts are formed in the degree that graft storing has no problem. On the other hand, a soft gate is separately prepared, and the gate and the hinge mechanism may be formed by only the soft gate. In addition, when the outer cylinder is molded with plastic, the hinge part and the opening and closing gate may be integrally molded.

Furthermore, if the opening and closing gate has the hinge mechanism, the direction of opening and closing can be selected in various directions. For example, the opening and closing gate may be opened and closed in the axis direction of the injector for an eye or in the perpendicular direction with respect to the axis direction.

Examples of the usage of the injector for an eye having the outer cylinder with the opening and closing gate are followings: when the gate is opened a graft is placed, and then the gate is closed to store the graft in the cylinder. In addition, when the gate is opened a graft is placed, a graft holding part of the graft holding structure described later is arranged on the graft, and then the gate is closed to store the graft to be rolled on the graft holding part.

In a common posterior lamellar keratoplasty, a graft usually has a diameter of 8 to 9.2 mm, and it is preferable that the graft storage part has a shape and a size for storing the graft without load as well as the opening and closing gate and the hinge mechanism. Furthermore, it is preferable that a lock or a clamp is equipped for fixing closing state when the gate is closed. In addition, it is preferable that the graft storage part has a structure in which when the gate is closed a large concave-convex is not formed on the inner surface for preventing graft damage.

Preferably, the injector for an eye of the invention has a stage for temporarily placing the graft around the graft storage part, for example near the aperture or the opening and closing gate of the outer cylinder, and especially adjacent to the aperture or the opening and closing gate. For example, an operator can use the stage for temporarily holding a graft while setting the graft to the graft storage part, or for preparing the graft to be rolled when the graft is needed to be rolled for setting to the graft storage part. If the stage is formed, when the graft is carried from a preparation base (for example, a cutting block) to the injector for an eye, the graft can be set into the injector for an eye in a comparatively minimally invasive way (especially, when an adjacent stage is formed, the graft can be slid for setting). For example, a cutting block where a graft is shaped is placed alongside the stage of the injector for an eye, and then the graft is slid to place on the stage. Preferably, the width of the stage is a range of 1 to 10 mm and the length is a range of 4 to 20 mm.

An inner shape of the part for setting the graft (the inner space part) of the outer cylinder is not specifically limited, and may be a shape and a size where a graft can be inserted in the outer cylinder. The inner shape of the inner space part may be for example trumpet shaped. The trumpet shape in the specification means that the inner shape of the inner space part of the outer cylinder formed in the injector for an eye for easily graft storing have the structure in which the width of the inner space part gradually increases from the leading end of the outer cylinder to the graft storage part (for example, a taper-like structure). Furthermore, the trumpet shape may continue to the graft storage part of the outer cylinder. The structure make an operation of placing the graft holding structure having the graft into the outer cylinder easy and can reduce an impact on the graft.

The shape of the leading end of the outer cylinder is not specifically limited if the injector can be smoothly inserted into an eyeball without centesis, but the leading end may have a sloping shape for easier insertion into an eye. Furthermore, the insertion diameter of the outer cylinder is preferably ϕ 1 to 5 mm, and even more preferably ϕ 2 to 4 mm. For insertion through a smaller incision, it is preferable that the wall thickness of the cylinder shape part of the leading end is as thin as possible. The strength of a material should be considered for keeping the strength of the leading end, but the thickness of the leading end is preferably 0.4 mm or below, and more preferably 0.2 mm or below.

It is preferable that the material of the outer cylinder is sterilizable. Examples for the material of the outer cylinder are plastic, metal (such as stainless steel), glass, ceramic, and silicone, and specifically preferably examples are transparent plastics with excellent internal visibility of the outer cylinder such as polypropylene, polycarbonate, polyamide, polymethyl methacrylate, polyester, polyethylene terephthalate, acrylic resin, and polyethylene.

Preferably the outer cylinder has a high internal visibility. An operator can check the graft stored in the outer cylinder to transplant the graft more safely. The transparent material may be arranged all over the outer cylinder or on a part of the outer cylinder, for example, like a window.

Meanwhile, a part of the leading end of the outer cylinder may be formed by using a soft material such as silicone in order to reduce damage to an eyeball and the like. Furthermore, as described later, the outer cylinder or the injector for an eye may be coated for high slidability of the graft and the graft may be pretreated with a viscoelastic material and the like.

Furthermore, as described later, the outer cylinder may include a safety apparatus for minimally invasive surgery. Preferably, for example, a mechanism for preventing over-insertion or a mechanism to control sliding of the graft holding structure is installed.

All of or a part of the outer cylinder, for example, a leading end for inserting into an eyeball, may be a detachable disposable cartridge from a hygiene point of view.

The graft holding structure in the invention means a structure which holds the graft and is placed inside the outer cylinder and slidable with respect to the outer cylinder (furthermore all of or a part of the structure may be rotatable with respect to the outer cylinder). Furthermore, the graft holding structure has a part where the graft directly touches (hereinafter referred to as a graft holding part). The graft holding part has important roles such as holding a graft shape or preventing graft reversion, storing the graft into the outer cylinder, pushing-out the graft from the outer cylinder, and discharging a fluid to the graft pushed-out from the outer cylinder. The shape of the graft holding structure is not specifically limited if the structure can be slidably installed in the outer cylinder, and may be shapes such as rod-like, spoon-like, and bowl-like. Furthermore, the cylinder shaped structure may have an expander movable by fluid pressure.

Furthermore, it is preferable that the graft holding structure includes a slide control lever for easy operations such as sliding in the outer cylinder.

The sliding in the specification means moving the graft holding structure back and forth along the axis direction of the outer cylinder in the inner space. Specifically, examples of the sliding are the following movements: a graft is placed on the graft holding part of the graft holding structure, and when the graft holding structure is advanced, the graft holding part holding the graft protrudes from the leading end of the outer cylinder or the outer cylinder to carry the graft which can be taken out from the outer cylinder; or the graft is pushed out form the outer cylinder. Specifically, the rotation is relative rotation of the outer cylinder with respect to the graft holding structure which is gripped or relative rotation of the graft holding structure with respect to the outer cylinder which is gripped.

The external diameter of the graft holding structure needs a size in which the graft holding structure can be stored in the outer cylinder and have a good slidability to the outer cylinder, and furthermore when the graft holding structure is stored in the outer cylinder, the graft can be stored in a space between the graft holding part formed in the graft holding structure and the inner surface of the outer cylinder. The external diameter in that case depends on the inner size (or the inner diameter) of the outer cylinder, but preferably ϕ 1 to 6 mm, and more preferably ϕ 2 to 5 mm.

Furthermore, it is preferable that the material of the graft holding structure is sterilizable. Examples of the material of the graft holding structure are plastic, metal (such as stainless steel), glass, ceramic, and silicone, and a specifically preferable material is selected from plastic and metal (such as stainless steel). For preventing a graft caught in the interface between the graft holding structure and the inner surface of the outer cylinder when the graft holding structure slides, a material adhering to the outer cylinder such as an O-ring or a rubber packing may be used for the interface, especially the interface having the possibility of graft touching. Furthermore, from a hygiene point of view, all of or a part of the adhering material may be a disposable cartridge detachable from the structure.

Furthermore, the graft holding structure may include a fluid discharge hole toward an inner surface of the graft taken out from the outer cylinder. The position of the discharge hole is not specifically limited if a fluid can be discharged to the inner surface of the graft, but in order to reliably deliver the graft to a transplant intended position, the discharge hole is preferably formed at the graft holding part which holds the graft.

As described above, the graft holding part has important roles such as holding a graft shape or preventing graft reversion, storing the graft into the outer cylinder, pushing-out the graft from the outer cylinder, and furthermore discharging a fluid to the graft pushed-out from the outer cylinder. The pushing-out the graft from the outer cylinder is delivery of the graft from the outer cylinder of the injector for an eye storing the graft into an eyeball. It also includes exposure of the graft, which is inserted into an eye by the injector for an eye, from the leading end of the outer cylinder of the injector for an eye to the anterior chamber.

An example of the graft holding structure includes a structure containing two cylinders of a middle cylinder and a inner cylinder, and specifically the inner cylinder is arranged in the middle cylinder and the inner cylinder is slidable with respect to the middle cylinder. Preferably each of the middle cylinder and the inner cylinder is independently slidable with respect to the outer cylinder, and more preferably is rotatable relative to the outer cylinder. The middle cylinder in the specification means the structure capable of being stored between the outer cylinder and the inner cylinder. The inner cylinder in the specification means the structure capable of being stored in the inner space of the middle cylinder. In other words, each of the external diameters is in the order of the outer cylinder > the inner cylinder > the inner cylinder, and preferably the respective cylinders are slidable to each other.

A more preferable example is the structure in which the inner cylinder is stored in the middle cylinder, a leading end of the inner cylinder protrudes a predetermined length (for example about 10 mm) from a leading end of the middle cylinder, a graft is placed on the protruded part (on the inner cylinder, a length of 10 mm), and the graft holding part is formed by the inner cylinder or by both the inner cylinder and the leading end face of the middle cylinder.

The external diameter of the middle cylinder is not specifically limited if the middle cylinder can be stored in the outer cylinder and has a good slidability with respect to the outer cylinder, but for example ϕ 2 to 5 mm. Furthermore, for preventing a graft caught in an interface when the middle cylinder slides relative to the other members, it is preferable that a material adhering to the inner or outer cylinder such as an O-ring or a rubber packing is used for the interface, especially the interface having the possibility of graft touching (for example, the leading end face of the middle cylinder).

Furthermore, in the embodiment, the wall thickness of the middle cylinder is not specifically limited if the space for storing and holding a graft between the outer cylinder and the graft holding part formed in the inner cylinder (or formed in both the inner cylinder and the leading end face of the middle cylinder) can be kept and the middle cylinder has a good slidability with respect to the outer cylinder and the inner cylinder, but for example 0.4 to 2.5 mm. Preferably, the middle cylinder has a slide control lever for easy operation such as sliding.

The external diameter of the inner cylinder is not specifically limited if the inner cylinder has a good slidability with respect to the middle cylinder, but for example ϕ 0.1 to 3 mm. Furthermore, preferably the inner cylinder has an inner space or a lumen for fluid flow, and in that case it is preferable that the external diameter of the inner cylinder is ϕ 0.3 to 3 mm and the inner diameter of the lumen is ϕ 0.1 to 1.5 mm.

The arrangement of the inner cylinder with respect to the middle cylinder may be the same axis as that of the middle cylinder or a different axis. For example, when the opening and closing gate of the graft storage part of the outer cylinder is opened to place a graft, the graft holding part formed in the inner cylinder is placed on the graft, and then the gate is closed to store the graft, the longer the distance between the graft holding part of the inner cylinder and the graft is, the less the touching risk is when the graft holding part of the inner cylinder is placed. Accordingly, it is sometimes preferable that the axis of the graft holding part of the inner cylinder is arranged at a distant position from the axis of the middle cylinder when viewed from the graft.

It is preferable that the middle cylinder and the inner cylinder have a safety apparatus for controlling sliding with respect to the outer cylinder, described later. Examples for the safety apparatus are a stopper mechanism, a lock mechanism, a frame mechanism, and a plate spring mechanism.

All of or a part of the middle cylinder and the inner cylinder may be detachable disposable cartridges from a hygiene point of view.

An illustrative example for graft pushing-out in the embodiment is the following procedure: a graft is stored in the injector for an eye including the outer cylinder, the middle cylinder, and the inner cylinder, with the leading end of the outer cylinder inserted through an corneoscleral incision into an eyeball, the middle cylinder and the inner cylinder are simultaneously slid and advanced with respect to the outer cylinder toward the inside of the eye, so that a part of the graft held on the graft holding part is protruded from the aperture of the leading end of the outer cylinder, then only the middle cylinder is still slid and advanced with respect to the outer cylinder and the inner cylinder to protrude all of the graft into the eyeball at the most leading end face of the middle cylinder, and the graft is exposed so as to be taken out from the outer cylinder. Other examples are the following procedures: with the leading end of the outer cylinder inserted through an corneoscleral incision into an eyeball, the middle cylinder and the inner cylinder are simultaneously slid and advanced with respect to the outer cylinder toward the inside of the eye, so that all of the graft held on the graft holding part is protruded into the eyeball and the graft can be taken out from the outer cylinder; and with the same condition as the above, only the middle cylinder is slid and advanced with respect to the outer cylinder and the inner cylinder, and all of the graft is protruded into the eyeball at the most leading end face of the middle cylinder, so that the graft can be taken out from the outer cylinder.

Furthermore, the inner cylinder or the middle cylinder in the embodiment may include a fluid discharge hole toward an inner surface of the graft taken out from the outer cylinder. It is desirable that the position of the discharge hole is arranged so as to discharge a fluid toward the inner surface of the graft, and in order to reliably deliver the graft to a transplant intended position, it is desirable that the discharge hole is arranged on the graft holding part of the inner cylinder holding the graft.

Furthermore, the discharge hole is desirable to be arranged so as to be under the central part of the graft when the graft is pushed-out form the outer cylinder into an eye. The arrangement can most easily reduce the possibility of turning over or folding the graft when the graft is lifted up and delivered to the transplant site by a fluid. Furthermore, since the rolled circumference part held on the graft holding part of the inner cylinder can deliver to a transplant site with spreading, it is very favorable for surgical technique. The central part of the graft in the specification is not specifically limited if the graft can be lifted up by fluid hitting without turning over and the like.

Preferably the discharge hole in the embodiment is, for example, formed within 6 mm from the end face of the leading end of the inner cylinder or the leading end of the inner cylinder. This is because the discharge hole comes under the central part of the graft when the graft is pushed-out into an eye to be taken out from the outer cylinder.

The shape of the discharge hole is not specifically limited. Furthermore, for example, since the edge of the discharge hole touches the graft when the discharge hole is arranged on the outer surface or the leading end of the inner cylinder, preferably the edge does not have burrs, and more preferably the edge is chamfered. Chamfering in the specification means that the edge of the discharge hole is not angulated and the corner is rounded. For example, after the hole on the cylinder is manufactured by cutting or laser, chamfering is performed by electro polishing or inserting a reamer into the hole. The diameter of the discharge hole is preferably 10 to 1000 µm, and more preferably 20 to 800 µm. Furthermore, the number of the discharge hole is optional, but preferably 10 or below, more preferably 5 or below, and even more preferably 1. The less the number of the discharge hole is, the more stable the fluid is likely to be able to be discharged, and the easier the graft uplift, namely delivering the graft to a transplant intended position, is likely to be.

Fluid feeding for discharging toward the inner surface of the graft in the invention can be performed by forming an inner space part in the graft holding structure and flowing a fluid in the inner space part. In the embodiment, the fluid may be discharged from the discharge hole through the inner space part in the inner cylinder. Furthermore, an additional cylinder shaped structure may be arranged in the inner space part of the graft holding structure, and in that case the fluid can be fed by using a space (a lumen) formed between the inner surface of the inner space part and the outer surface of a tubular structure.

For the fluid both liquid and gas may be used. The liquid is not specifically limited if used in the ophthalmologic field, and examples are intraocular infusion, saline, a Ringer's solution, dextrose in water, an injection fluid, a buffer, a viscoelastic solution, silicone oil, and perfluorocarbon. The gas is not specifically limited if used in the ophthalinologic field, and examples are sterilized air, nitrogen, and expandable gas (SF₆, C₃F₈). Both the liquid and the gas fulfill the roll, but from the viewpoint of safety and efficacy the gas is preferable and the sterilized air is more preferable.

It is preferable that the injector for an eye of the invention has a mechanism for controlling an intraocular pressure at the time of injector insertion or for forming the anterior chamber. The mechanism prevents reduction of the intraocular pressure or contraction of the anterior chamber caused by for example flowing out of an intraocular fluid from a corneoscleral incision when the injector for an eye is inserted into an eyeball through the corneoscleral incision. Specifically, the injector for an eye includes a discharge hole for liquid (intraocular infusion), and preferably the intraocular infusion is discharged from the graft holding structure. In the case of the embodiment in which the graft holding structure includes the inner cylinder and the middle cylinder, the intraocular infusion is discharged from a void (an aperture) formed between the leading end or the side face of the inner cylinder or the middle cylinder, or the outer surface of the inner cylinder and the inner surface of the middle cylinder. The void with a very small size is not preferable, because the intraocular infusion cannot be discharged from a bottle containing the intraocular infusion by the gravity.

It is preferable that feeding a liquid for controlling intraocular pressure to the liquid discharge hole is performed from the inner space part formed in the graft holding structure (in the case of the above embodiment, the inner cylinder or the middle cylinder). When a tubular structure is installed in the inner space part, the liquid may be fed from a hollow portion (a lumen) formed by an inner surface of the tubular structure or by using a space (a lumen) formed between the inner surface of the inner space part and an outer surface of the tubular structure.

The discharge hole for discharging a fluid toward the inner surface of the graft and the discharge hole for discharging the intraocular infusion may be the same hole, but may be different for a safer and more reliable operation.

It is preferable that the injector for an eye of the invention includes a mechanism for relieving the pressure in an eyeball (an intraocular pressure). The mechanism for relieving the intraocular pressure in the invention means a mechanism for reducing the high intraocular pressure when a graft is lifted up with a fluid and delivered to a transplant site and/or the high intraocular pressure caused by the liquid which is discharged in order to keep an intraocular pressure. It is not preferable that the injector for an eye is removed with a high intraocular pressure, because the graft delivered to the affected area becomes unstable by the pressure decrease. Sometimes, the graft which is delivered to a transplant site to be fixed moves from the transplant site or is peeled from the transplant site.

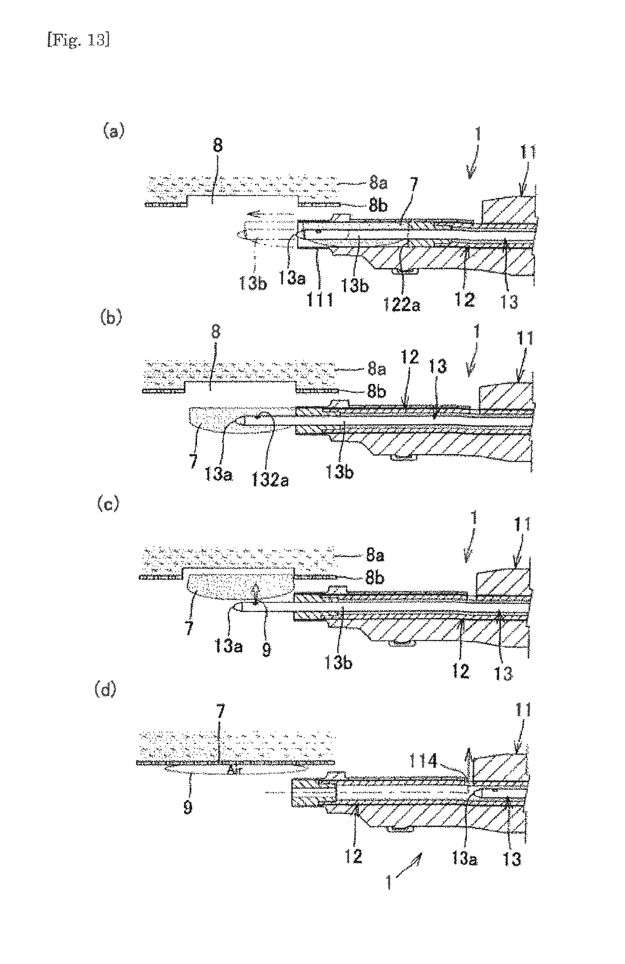

The mechanism is not specifically limited if an intraocular pressure can be relieved, may be the method in which the graft holding structure (in the case of the above embodiment, the middle cylinder or the inner cylinder included in the graft holding structure) is slid toward the outside of the eyeball, or the perfusion lumen for keeping the intraocular pressure is opened to relieve the intraocular pressure, and may be a pressure outlet which is formed on the graft holding structure (in the case of the above embodiment, the middle cylinder included in the graft holding structure) or the outer cylinder and communicates with outside of an eyeball. In addition, these methods may be properly combined. The shape and the structure of the pressure outlet in the specification are not specifically limited and may be the proper size and the number for relieving an intraocular pressure. An example of the preferred embodiments is shown in Figure 13 (d). After separation and expansion of the graft, the inner cylinder 13 is slid toward the outside of the eyeball to the pressure outlets 114 (circular holes with a width of 1 mm) which are installed 2 cm away from the leading end of the middle cylinder 12 and the outer cylinder 11, and the aperture of the leading end of the outer cylinder (and the middle cylinder) communicates with the aperture (the pressure outlet 114) formed on the side face of the outer cylinder (and the middle cylinder), so that the passage (in Figure, shown by the arrow) from the inside of the eyeball toward the outside is kept.

The injector for an eye of the invention may have a surface with lubricious coating. The lubricious coating in the specification means, for example, coating on the inner surface of the outer cylinder for high slidability of a graft mainly in the outer cylinder or on the inner cylinder. The type of the coating is not specifically limited if the object is achieved, and may be hydrophilic coating, hydrophobic coating, smooth coating, polymer coating, metal coating, ceramic coating, and the like. Examples for preferred hydrophilic coating materials are the following synthetic or natural polymers or derivatives thereof. polyethylene oxide polymers (for example, polyethylene glycol and derivatives of polyethylene glycol and urethane), maleic anhydride polymers (for example, maleic anhydride copolymers such as methyl vinyl ether-maleic anhydride copolymer), acrylamide polymers (for example, N,N-dimethyl acrylamide), water-soluble nylons, polyvinylpyrrolidone polymers, hydroxyl group-containing polymer (for example, polyvinyl alcohol and 2-hydroxylethyl methacrylate), cellulose polymers (for example, hydroxylpropyl cellulose), hyaluronic acid, chondroitin, chondroitin sulfate, keratan sulfate, keratan polysulfate, heparin acid, and mucopolysaccharides thereof, and alginic acid and salts thereof. These compounds are preferred because a low coefficient of friction is stably obtained. Polysaccharides are preferred because of high safety for medical use. Treatments such as surface plasma are also effective.

Preferred hydrophobic coating materials are fluorine compounds and silicone compounds. Preferred metal coating materials are titanium compounds (for example, titanium oxide). Preferred ceramic coating materials are alkoxy metal salts and aqueous metal salts.

Furthermore, the injector for an eye of the invention may include a mechanism for preventing over-insertion. The mechanism for preventing the over-insertion is a safety apparatus for minimally invasive surgery. That is, it is preferable that the safety apparatus is installed in the outer cylinder and the graft holding structure (in the case of the above embodiment, the middle cylinder and the inner cylinder included in the graft holding structure) in order to prevent the over-insertion into an eyeball from occurring. For example, in the case of the prevention of the outer cylinder over-insertion, it is preferable that a posterior part to a predetermined distance from the insertion leading end of the outer cylinder is made thicker than the incision or a thicker part (a pod) or a flange is installed. For example, when an incision is 5 mm, the outer cylinder within 3 mm from the leading end makes an external diameter of ϕ 3 mm and the outer cylinder from that point makes an external diameter of ϕ 7 mm. In the case of the prevention of the over insertion of the graft holding structure (in the case of the above embodiment, the middle cylinder included in the graft holding structure), for example, a slide control lever may be installed in the structure and the lever hits to a stopper or a wall installed on the outer cylinder not to move any further. Furthermore, the prevention of the over-rotation is the same way. Furthermore, in the above embodiment in which the graft holding structure includes the inner cylinder and the middle cylinder, for the prevention of the over-insertion of the inner cylinder, for example, an operation apparatus (for example, a luer lock connector) may be attached on a posterior part of the inner cylinder and the apparatus hits to the side face of the grip part of the outer cylinder not to move any further.

Manners for controlling slidability between the outer cylinder and the graft holding structure (in the case of the above embodiment, between the outer cylinder, the middle cylinder, and the inner cylinder) of the injector for an eye of the invention are not specifically limited, but the slidability can be controlled by, for example, the method using elasticity such as a rubber packing, an O-ring, and a tube, each cylinder diameter (compliance), lubricating liquid (for example silicone oil), a screw, a plate spring, or combinations thereof. The slidability in the specification means easy control for an operator in which specifically when a force is applied by a finger, each of the cylinders and the structure moves, and when a force is not applied, each does not move. In the above embodiment in which the graft holding structure includes the inner cylinder and the middle cylinder, specific examples are the followings: the slidability between the inner cylinder and the middle cylinder is controlled by tube elasticity (for example, a thin tube is fixed outside the inner cylinder), and the slidability between the middle cylinder and the outer cylinder is controlled by the elasticity of a rubber packing installed on one end of the middle cylinder (for example, a syringe pusher). The outer cylinder and the inner cylinder may be controlled by the elasticity of a rubber packing installed on one end of the middle cylinder.

A bottle containing the intraocular infusion and the injector for an eye may be connected via a tube and the like. For example, a connector with a luer lock (for example, a Y-shaped connector, a V-shaped connector, a 2-way connector, and a 3-way cock) connected to the graft holding structure (in the case of the above embodiment, the inner cylinder included in the graft holding structure) may be used. Furthermore, a fluid discharge apparatus for lifting up a graft with a fluid and delivering to a transplant site (for example, a syringe or a pump) and the injector for an eye may be similarly connected via a tube and the like.

The amount of the fluid poured from the fluid discharge apparatus is not specifically limited, it is not preferable that the amount is too little, because a graft cannot be thoroughly lifted up, and it is not preferable that the amount is too much because of the safety issue for an eye. A preferred example is slow pouring of 0.05 to 1 mL of clean air into an eye. At that time, touching the fluid discharge apparatus to near the center of the graft, smooth delivery to the transplant site (the affected area) can be achieved.

Hereinafter, the present invention will be further described in detail with reference to the embodiments shown in the drawings, but the invention is limited by the embodiments.

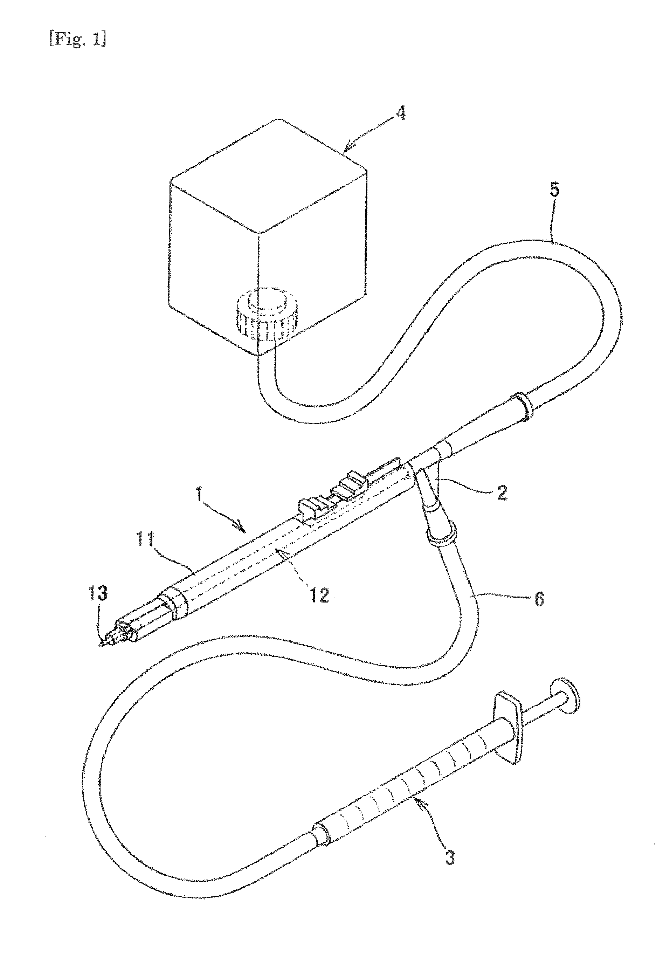

Figure 1 shows an example for an ophthalmic medical device set using the injector for an eye according to the present invention. The medical device set includes an injector for an eye 1 of a first embodiment, a Y-shaped connector 2 connected to an edge part near to hand of a grip part of the injector for an eye 1, a syringe 3 which is connected to the Y-shaped connector 2 via a tube 6 and is a fluid discharge apparatus for lifting up and delivering a graft taken out from an outer cylinder included in the injector for an eye to a transplant site, and a bottle 4 which is connected to the Y-shaped connector 2 via a tube 5 and contains intraocular infusion in order to keep an intraocular pressure.

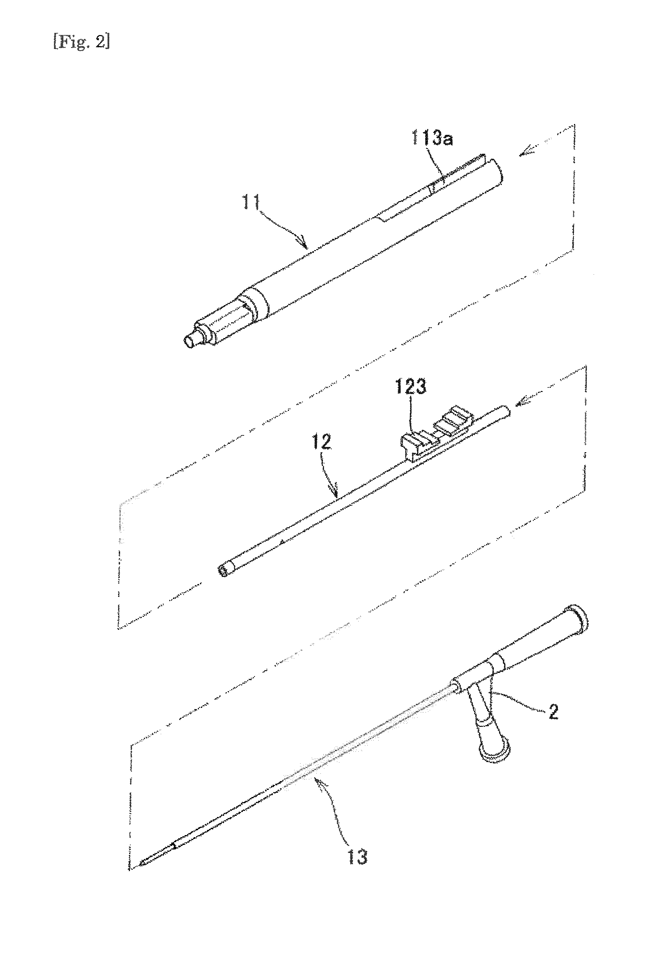

Figure 2 is an exploded view showing an outer cylinder 11, a middle cylinder 12, and an inner cylinder 13 included in the injector for an eye 1 according to the invention. As shown by the arrows in Figure 2, the inner cylinder 13 installed with the Y-shaped connector 2 is inserted into the middle cylinder 12, and then the middle cylinder 12 as such is inserted into the outer cylinder 11 to assemble the injector for an eye 1 as shown in Figure 2.

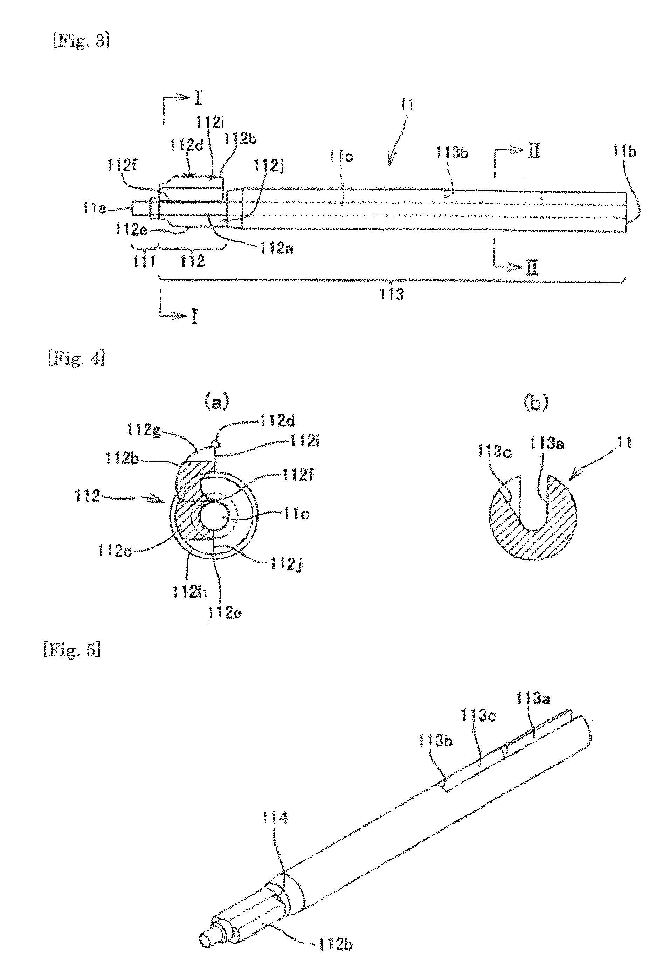

Figure 3 is a side view showing the outer cylinder 11 of the embodiment (with a gate open), Figure 4 (a) is a cross-sectional view taken along line I-I in Figure 3, Figure 4 (b) is a cross-sectional view taken along line II-II in Figure 3, and Figure 5 is a perspective view of the outer cylinder 11 (with the gate closed). As shown in Figure 3, the outer cylinder 11 includes a leading end 111 and a grip part 113 and has an approximately cylinder-shaped structure from a distal end 11a of the leading end 111 to a proximal end 11b of the grip part 113. It is preferable that the outer cylinder 11 is made of a material with visibility to an inner space 11c for graft observation such as transparent plastic. Furthermore, it is preferable that a surface of the inner space 11c of the outer cylinder 11 is coated with hydrophilic coating.

The leading end 111 has a cylindrical-shaped structure with thin-wall to be suited for the insertion into an eyeball.

A graft storage part 112 of the grip part 113 is formed adjacent to the leading end 111, the graft storage part 112 is halved in the axis direction of the outer cylinder 11 to form an aperture 112a, the aperture 112a can be opened and closed by a hinged gate 112b with a hinge structure, the graft storage part 112 includes a fit fixing part 112c of the outer cylinder 11 together with the gate 112b, and the fit fixing part 112c has an opening edge 112h which is installed with locks (112d, 112e) for closing the gate 112b by fitting concave and convex of the locks (112d, 112e) each other. The length of the gate 112b along the axis direction of the outer cylinder 11 makes shorter than that of the aperture 112a along the same direction, and as shown in Figure 5, a pressure outlet 114 is formed at the side of the grip part 113 so that an inner space 11c would communicate with the outside when the gate 112b is closed.

An opening edge 112g of the gate 112b in the graft storage part 112 and an aperture edge 112h of the fit fixing part 112c opposed to the opening edge 112g have planar stages 112i and 112j, respectively, and the planar stages 112i and 112j can abut each other.

The grip part 113 has a groove 113a for sliding an operational bar 123 formed on the middle cylinder 12, and the groove 113a has a predetermined length from a proximal end 11b of the outer cylinder 11 toward a distal side 11a along the axis direction. The groove 113a has a distal end (a dead end) 113b which works as a stopper for preventing the over-insertion by hitting the operational bar 123 of the middle cylinder 12 to the distal end 113b. Furthermore, a lock mechanism is installed for fixing the operational bar 123. The lock mechanism is a notch structure (113c) in the direction of the outer cylinder rotation and the length of the notch structure (113c) from the distal end (the dead end) 113b of the groove 113a is the length of the operational bar in the axis direction of the outer cylinder.

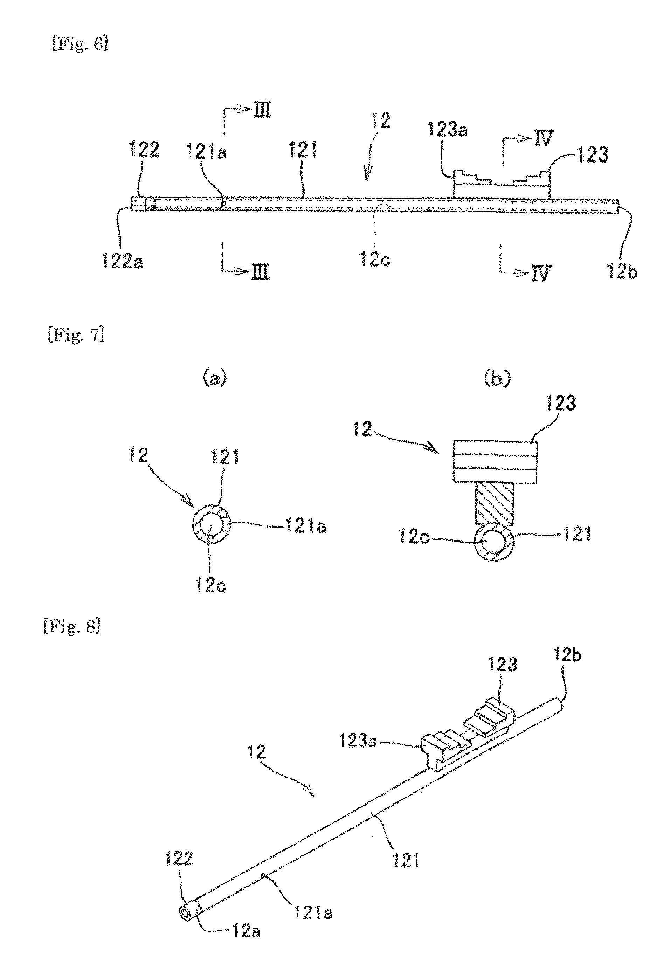

Figure 6 is a side view showing the middle cylinder 12 of the embodiment, Figure 7 (a) is a cross-sectional view taken along line III-III in Figure 6, Figure 7 (b) is a cross-sectional view taken along line IV-IV in Figure 6, and Figure 8 is a perspective view of the middle cylinder 12. As shown in Figure 6, the middle cylinder 12 includes a cylindrical-shaped tube 121, an approximately cylindrical-shaped packing 122 formed at a leading end (a distal end) 12 of the middle cylinder 12, and an operational bar 123. The tube 121 has an inner space 12c throughout its entire length from the distal end (the leading end) 12a to a proximal end 12b and has an outlet 121a at a predetermined position from the distal end 12a. In the embodiment, when the position of the operational bar 123 is used as the standard, the outlet 121a is installed at 90° displaced position in the circumferential direction in the opposite direction from the notch structure (113c) as the lock mechanism of the outer cylinder 11 when the middle cylinder 12 is set in the outer cylinder 11 (Figure 7).

The packing 122 formed at the distal end 12a of the middle cylinder 12 has an approximately cylindrical-shaped structure, is slidable with respect to an inner perimeter of the outer cylinder 11 and an outer periphery of the inner cylinder 13, and also can keep the injector hermetic. Furthermore, the operational bar 123 is provided at a predetermined axial distance from the proximal end 12b of the middle cylinder. A side face of the distal end 123a of the operational bar 123 hits the end part (the dead end) 113b of the groove 113a of the outer cylinder 11 to prevent the over-insertion of the middle cylinder 12. The shape of the operational bar 123 has a stepped cross section shown in Figure 6 for easy finger operation.

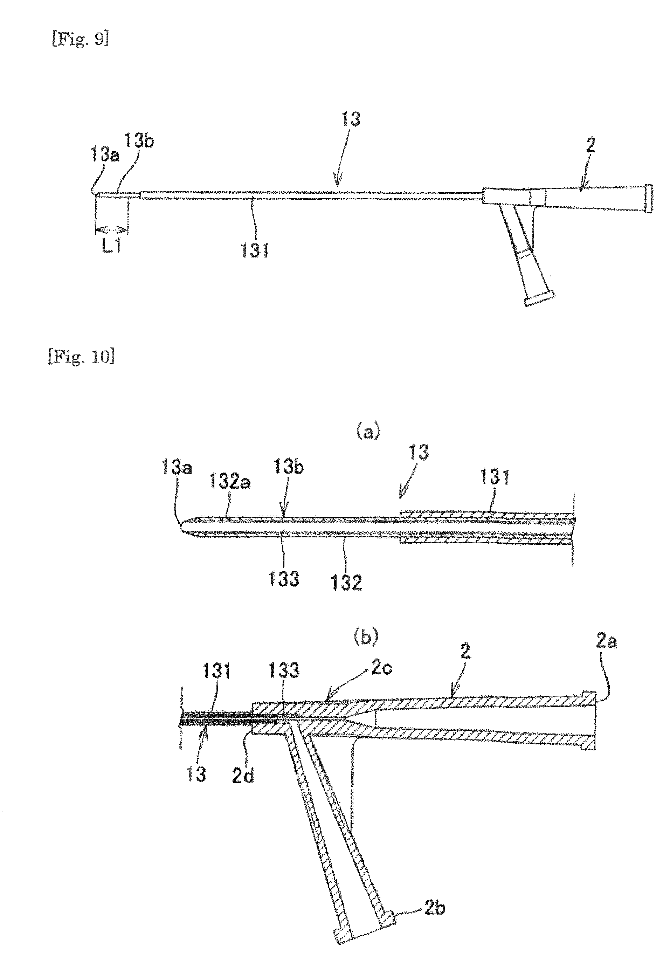

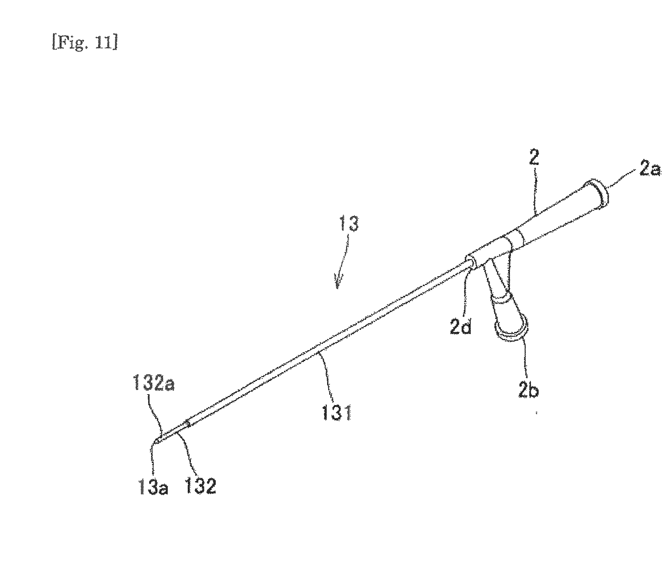

Figure 9 is a side view of the inner cylinder 13 of the embodiment and the Y-shaped connector 2 is installed. Figure 10 (a) is a sectional view of a distal end proximal portion of the inner cylinder 13, Figure 10 (b) is a sectional view of a proximal side (a base end) of the inner cylinder 13 and the installed Y-shaped connector 2, and Figure 11 is a perspective view of the inner cylinder 13 installed with the Y-shaped connector. As shown in Figure 10, the inner cylinder 13 includes an outer tube 131, a fluid discharge tube 132, and a perfusion tube 133 in the fluid discharge tube 132, and each has a cylinder-shaped structure. The Y-shaped connector 2 includes a perfusion port 2a and a fluid discharge port 2b, a distal part 2c of the Y-shaped connector 2 connects a perfusion tube 133 of the inner cylinder 13 so as to communicate with the perfusion port 2a, and connects the fluid discharge tube 132 so as to interpolate the perfusion tube 133 and to communicate with the fluid discharge port 2b (here, in order to help to understand the structure, no cross section of the perfusion tube 133 is shown in Figure 10). In this manner, a perfusion lumen is formed from the perfusion port 2a to the distal end (the leading end) 13a of the inner cylinder 13 through the perfusion tube 133 in the inside.

A graft holding part 13b is formed from the distal end (the leading end) 13a of the inner cylinder 13 to a predetermined position of the proximal side in the axis direction of the inner cylinder (L1), and the graft holding part 13b includes a discharge hole 132a in the embodiment. The discharge hole 132a communicates with the fluid discharge port 2b through a space between an outer periphery surface of the perfusion tube 133 and an inner perimeter of the fluid discharge tube 132. Furthermore, the over-insertion of the inner cylinder 13 can be prevented by hitting a side face of a distal end 2d of the Y-shaped connector to a side face of the proximal end 11b of the outer cylinder 11 or the proximal end 12b of the middle cylinder 12.

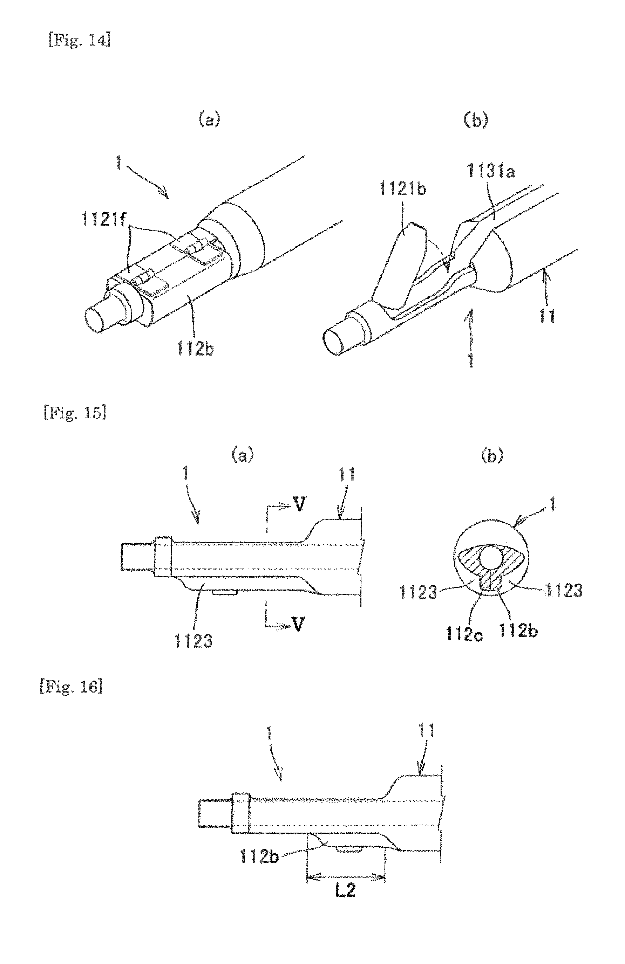

In addition to the embodiment, as shown in Figures 14 to 16, the injector for an eye 1 according to the invention may have other embodiments.

As shown in Figure 14 (a), the hinge mechanism may include hinges 1121f.

Furthermore, as shown in Figure 15, a part of the gate 112b and a peripheral part of the fit fixing part 112c which form the stages may have a hollow 1123 for easy finger gripping when closing the gate 112b.

Furthermore, in order not to abut the injector for an eye 1 to a patient face during an operation, as shown in Figure 16, the length (L2) of the stages 112i and 112j along the axis direction of the outer cylinder 11 may be shortened without losing the function of the stages.

Furthermore, the injector for an eye according to the invention may be the embodiment shown in Figure 14 (b). In the embodiment, the grip part 113 of the outer cylinder 11 has a groove 1131a with a predetermined width through almost the entire length of the axis direction of the outer cylinder. Furthermore, the gate 1121b is opened and closed in a different direction from that of the gate 112b in Figure 3 or 14(a) and the gate 1121b is turned in the axis direction of the outer cylinder 11 to be opened and closed.

Next, the usage of the embodiment will be explained. In Figure 13, for convenience of explanation, the outlet 121a of the middle cylinder 12 is shown at a different position from in Figure 6.

As shown in Figure 2, the middle cylinder 12 is inserted into the outer cylinder 11 and the inner cylinder 13 (the graft holding part 13b with a length of 9 mm is illustrative) is inserted into the middle cylinder 12 to assemble the injector for an eye 1. As shown in Figure 1, the bottle 4 containing an intraocular infusion and the syringe 3 are connected to the Y-shaped connector 2 installed on the inner cylinder 13 through the tubes 5 and 6, respectively. Not shown in Figure, cocks may be connected to the perfusion port 2a and the fluid discharge port 2b of the Y-shaped connector 2 to control flowing fluids for perfusate and discharge into an eye.

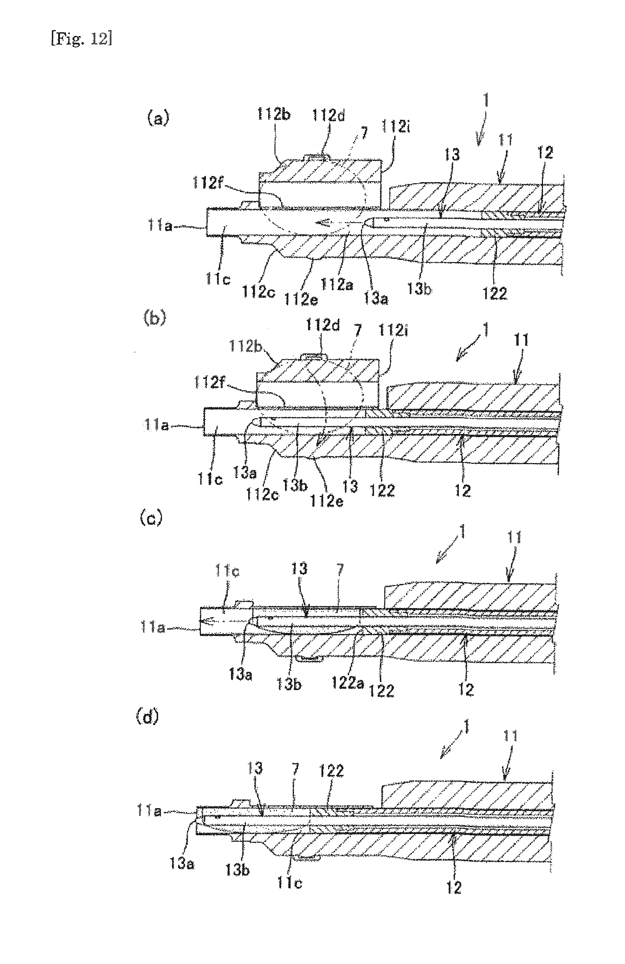

Next, as shown in Figure 12 (a), the gate 112b of the outer cylinder 11 is opened, the distal end 13a of the inner cylinder 13 is slid near the proximal end of the aperture 112a, and then the graft 7 with a diameter of 9 mm is placed on the hinge aperture (the inner space 11c formed in the fit fixing part 112c of the outer cylinder 11, the hinge part 112f, and the stage 112i of the gate 112b). The graft 7 is placed so that the endothelial face would contact the graft holding part 13b. At this time, the graft 7 may be coated with hyaluronic acid. Then, the inner cylinder 13 and the middle cylinder 12 are slid to the distal end side as shown by the arrow in Figure 12 (a). Next, as shown in Figure 12 (b), the gate 112b is closed so that the graft 7 would be rolled on the graft holding part 13b of the inner cylinder 13 (when hyaluronic acid is used, hyaluronic acid works as a glue to weakly bind the endothelial face of the graft 7 with the graft holding part 13b). As shown in Figures 12(c) and (d), the gate 112b is locked, the inner cylinder 13 and the middle cylinder 12 is simultaneously and slowly slid to near a distal end 11a of the inner cylinder with respect to the outer cylinder 11 to complete the preparation before insertion (the graft 7 is pushed by the inner cylinder 13 weakly bound by hyaluronic acid and the side face of the distal end 122a of the middle cylinder. At this time, the shape of the graft 7 is held by support of the inner cylinder 13 and sliding of the surface of the inner space 11c of the outer cylinder).

On the other hand, before the injector for an eye 1 in the above state is inserted into an eyeball, a needless inner layer of the cornea (the corneal stroma 8a, the corneal endothelium and the Descemet's membrane 8b) at a transplant intended position 8 in the eyeball is pre-peeled. Then, as shown in Figure 13 (a), the leading end 111 of the outer cylinder of the injector for an eye 1 is inserted 3 mm through the incision not shown in Figure (in this manner, the step in which the outer cylinder is inserted into an eyeball in the state that among the middle cylinder 12 and the inner cylinder 13 constituting the graft holding structure stored in the outer cylinder 11, the inner cylinder 13 stores a graft 7between the graft holding part 13b of the inner cylinder 13 and the inner surface of the outer cylinder 11, the step of inserting the outer cylinder into an eyeball is performed), and the middle cylinder 12 and the inner cylinder 13 are simultaneously slid 4 mm with respect to the outer cylinder 11 (simultaneously, the inner cylinder 13 and the side face of the distal end 122a of the middle cylinder physically push the graft 7 by 4 mm and the inner cylinder 13 comes out 4 mm from the outer cylinder 11). Then, as shown in Figure 13 (b), only the middle cylinder 12 is slid further 5.5 mm with respect to the outer cylinder 11 and the inner cylinder 13 (simultaneously, the side face of the distal end 122a of the middle cylinder pushes the graft 7 by 5.5mm and the middle cylinder 12 comes out 0.5 mm from the outer cylinder 11), the graft 7 with a diameter of 9 mm is taken out from the outer cylinder 11 to insert into the eye (in this manner, the step in which the inner cylinder 13 and the middle cylinder 12 constituting the graft holding structure is advanced with respect to the outer cylinder 11 and the graft 7 held on the graft holding part 13b is taken out from the outer cylinder 11 is performed). Next, as shown in Figure 13 (c), from the syringe 3 shown in Figure 1, 0.05 to 1 mL of clean air is slowly injected into the eyeball, the air 9 exhausted from the discharge hole 132a lifts up the graft 7 from the graft holding part 13b, and the graft 7 is delivered to the predetermined transplant intended position 8 as shown in Figure 13 (d) (in this manner, the step in which the air 9 as the fluid is discharged from the graft holding part 13b to the inner surface of the graft 7 taken out from the outer cylinder 11 is performed). Then, the distal end 13a of the inner cylinder 13 is slowly slid to reach the position of the pressure outlet 114 of the outer cylinder 11, an intraocular pressure is relieved, and then the injector for an eye 1 is removed from the eyeball.

During an operation, in order to keep an intraocular pressure or to form the anterior chamber, an intraocular infusion is properly perfused from the distal end 13a of the inner cylinder starting from the bottle 4 through the perfusion port 2a of the Y-shaped connector 2 and the perfusion tube 133 of the inner cylinder 13. Here, if the flow speed of the intraocular infusion is not proper, sometimes the graft movement in an eyeball becomes unstable, so that after the anterior chamber is formed by the injector for an eye, perfusion is sometimes preferable to be stopped.

In addition to the above method for inserting a graft into an eye, the following methods can be performed: after inserting the leading end 111 of the injector for an eye, the middle cylinder 12 and the inner cylinder 13 are slid 2 mm with respect to the outer cylinder 11 (simultaneously, the graft holding part 13b of the inner cylinder 13 and the side face of the distal end 122a of the middle cylinder physically push the graft 7 by 2mm, and the inner cylinder 13 comes out 2 mm from the outer cylinder 11), and next, only the middle cylinder 12 is slid 7.5 mm with respect to the outer cylinder 11 and the inner cylinder 13 (simultaneously, the side face of the distal end 122a of the middle cylinder pushes the graft 7 by 7.5mm, and the middle cylinder 12 comes out 0.5 mm from the outer cylinder 11); and after inserting the injector for an eye, only the middle cylinder 12 is slid 9.5 mm with respect to the outer cylinder 11 and the inner cylinder 13 (simultaneously, the side face of the distal end 122a of the middle cylinder pushes the graft 7 by 9.5mm, and the middle cylinder 12 comes out 0.5 mm from the outer cylinder 11).

In this manner, by adopting the injector for an eye according to the invention and the method for inserting a graft into an eyeball thereby, a graft can be inserted into an eyeball without any damage, and easily and reliably delivered to a desired transplant site, so that the injector for an eye according to the invention and the method for inserting a graft into an eyeball thereby can be suitably used for a method for eye treatment.

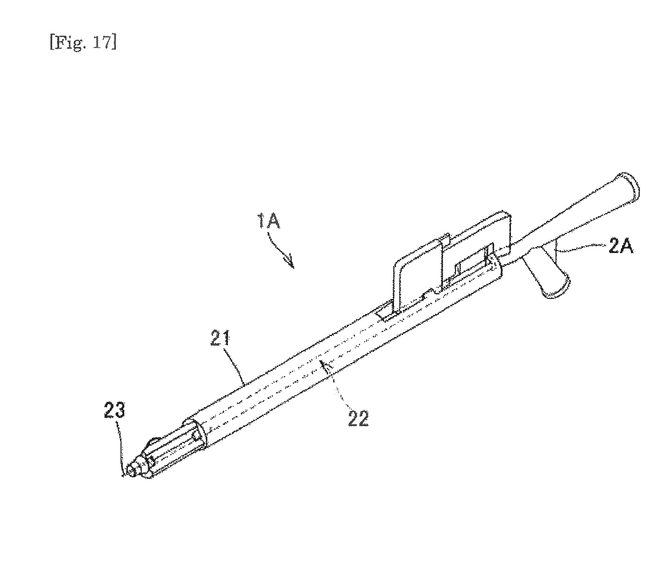

Figures 17 to 29 show the embodiment of the injector for an eye 1A and components thereof according to a second embodiment of the invention. Here, the same explanation as the first embodiment will be omitted.

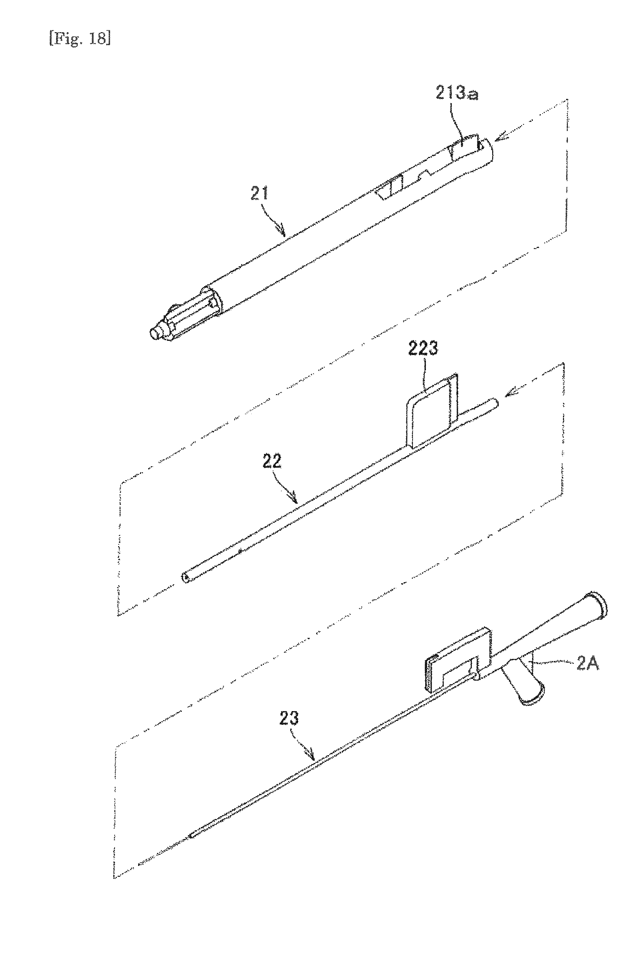

Figure 17 is a perspective view showing the injector for an eye 1A, Figure 18 is an exploded perspective view of the injector for an eye 1A showing the embodiment of each component. It is the same as in the first embodiment that respective components are assembled according to the arrows in Figure 18 to become the injector for an eye 1A showing in Figure 17.

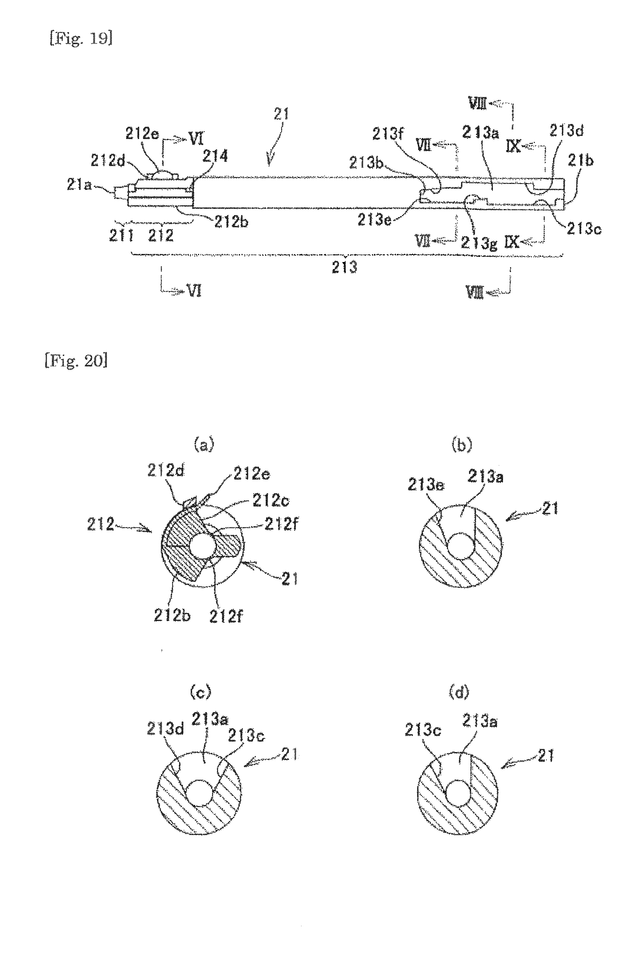

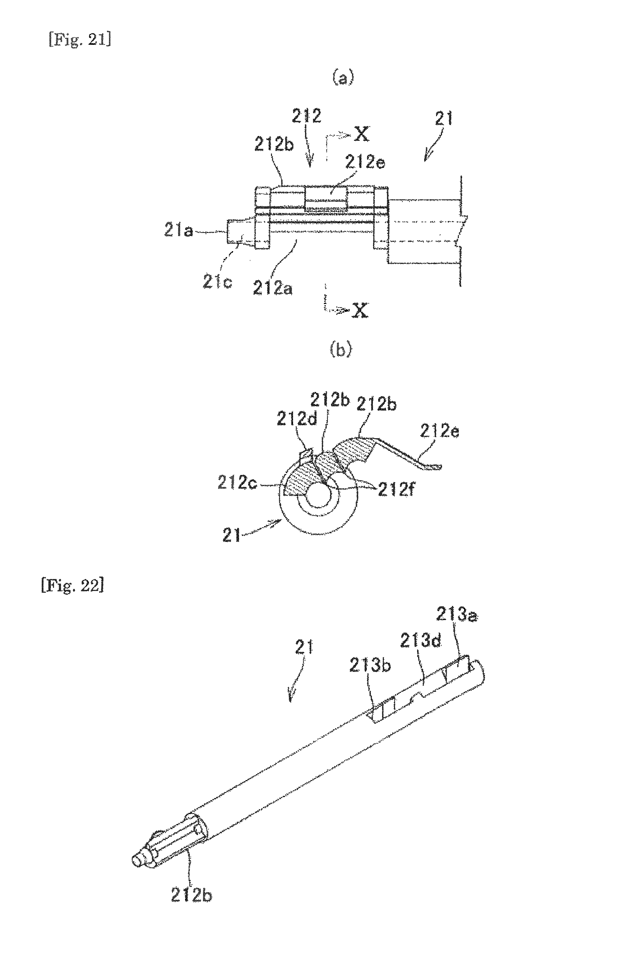

Figure 19 shows a top view of the outer cylinder 21 in the embodiment and shows the state in which the gate 212b formed on the aperture 212a in the graft storage part 212 is closed. Figure 20 (a) is a cross-sectianal view taken along line VI-VI of the graft storage part of the outer cylinder 21 shown in Figure 19, Figure 20 (b) is a cross-sectional view taken along line VII-VII in Figure 19, Figure 20 (c) is a cross-sectional view taken along line VIII-VIII in Figure 19, and Figure 20 (d) is a cross-sectional view taken along line IX-IX in Figure 19. Figure 21 (a) is an enlarged fragmentary view of the leading end side of the outer cylinder 21 with the gate 212b open, and Figure 21 (b) is a cross-sectional view taken along line X-X in Figure 21 (a). Figure 22 is a perspective view of the outer cylinder 21.

A basic structure of the outer cylinder 21 in the embodiment is common to the outer cylinder 11 in the first embodiment, but the outer cylinder 21 has different features described below: the aperture 212a can be opened and closed by the gate 212b which has a hinge structure and is formed by three-division of a circumference of the outer cylinder; and the grip part 213 has any of the notch structures 213c to 213g different from that in the first embodiment.

In this manner, since the aperture 212a can be opened and closed by the three-divided gate 212b with the hinge structure (hinge parts 212f), when the gate is opened (see Figure 21 (b)), the three-divided gate (Figure 21 (b)) has a smaller curvature than that of the two-divided gate (see Figure 4 (a)), thus the total length of arc chords becomes large, so that the graft 7 is easily placed on the inner surface of the gate 212b, liquid or air is difficult to get into between the inner surface of the gate 212b and the graft 7, and the visibility for the graft 7 improves.

Furthermore, when the notch structures 213c to 213g of the outer cylinder 21 shown in Figure 19 and the like are combined with an operational bar 223 and an inner cylinder operational bar 24 respectively installed in the middle cylinder 22 and the inner cylinder 23 described later, the middle cylinder 22 and the inner cylinder 23 can slide more safely with respect to the outer cylinder 21.

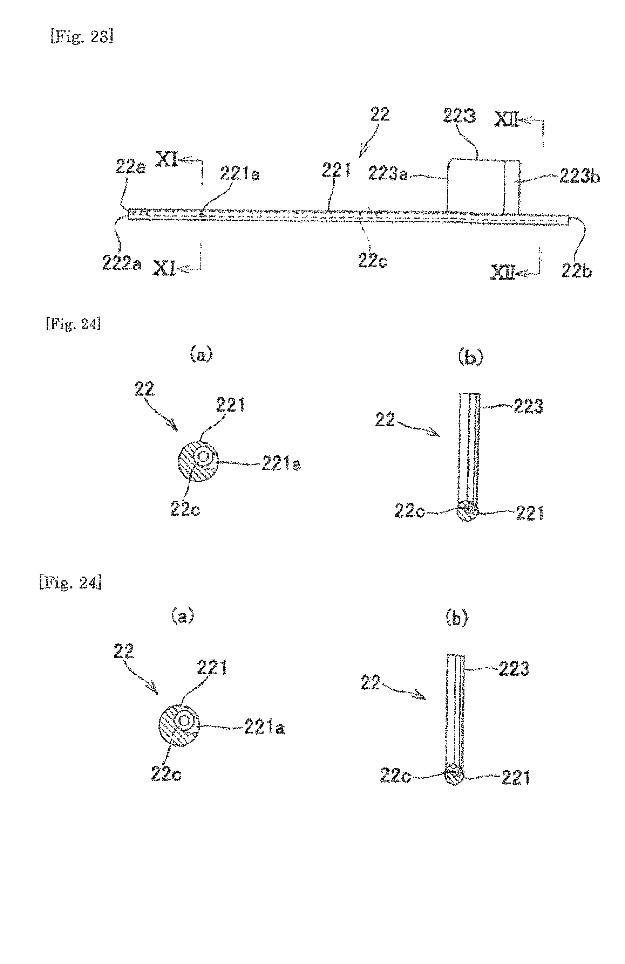

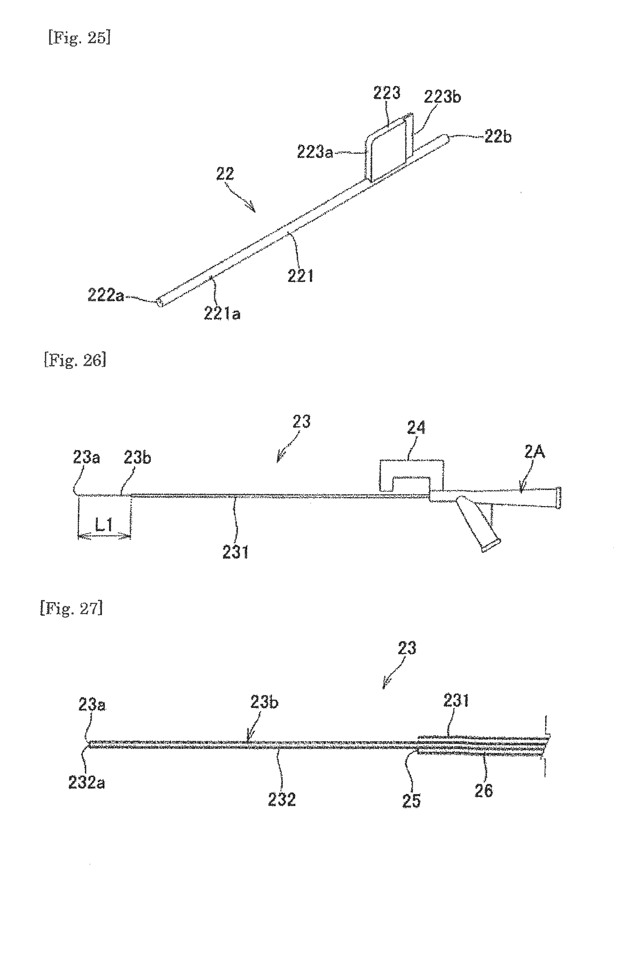

Figure 23 shows a side view of the middle cylinder 22 in the embodiment and the shape of the inner space 22c is shown by broken lines. Figure 24 (a) is a cross-sectional view taken along line XI-XI in Figure 23, and Figure 24 (b) is a cross-sectional view taken along line XII-XII in Figure 23. Figure 25 is a perspective view of the middle cylinder 22.

The middle cylinder 22 in the embodiment is a cylinder-shaped tube having the operational bar 223, the distal end (the leading end side) 22a communicates with the proximal end (the rear end side) 22b, the sizes of the leading end side and the rear end side of the inner space 22c of the middle cylinder 22 (the inner diameter) are different, and the size of the leading end side is thinner than that of the rear end side. Furthermore, the leading end side and the rear end side of the inner space 22c are formed coaxially, but the axis is arranged not to correspond to the central axis of the middle cylinder 22. This is for keeping the distance between the leading end of the inner cylinder 23 to fit to the inner space 22c of the middle cylinder 22 and the graft 7 placed on the gate 212b of the outer cylinder 21, and for less damage to the graft 7 when the inner cylinder 23 is advanced on the graft 7. The operational bar 223 is sequentially fitted to the notch structures 213c to 213g of the outer cylinder 21 described above to control advancing of the middle cylinder 22. Furthermore, the operational bar 223 is removably bound to the inner cylinder operational bar 24 installed on the inner cylinder 23 described later and the middle cylinder 22, and the inner cylinder 23 becomes one-body to control their advancing and retracting. In the example, a convex part 223b is formed on the rear end side of the operational bar 223 so as to fit a concave part 24a formed on the leading end side of the inner cylinder operational bar 24.

Furthermore, the middle cylinder 22 has the aperture on the side face 222a of the distal end of the middle cylinder in the same manner as the first embodiment, and has the outlet 221a for relieving an intraocular pressure so as to communicate with the aperture.

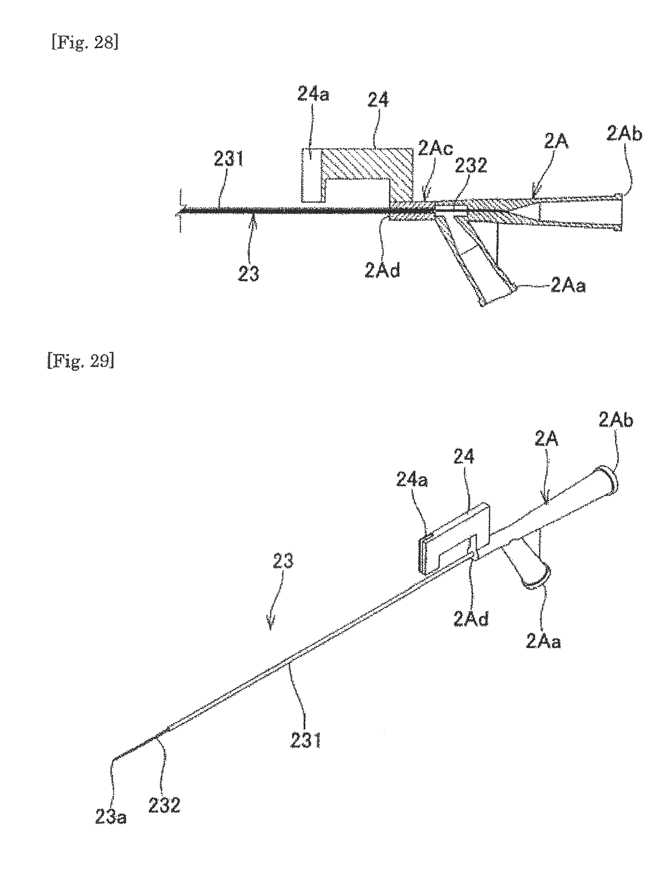

Figure 26 shows a schematic side view of the inner cylinder 23 in the embodiment. Figure 27 is an enlarged sectional view of the leading end of the inner cylinder 23, and Figure 28 is an enlarged sectional view of the rear end of the inner cylinder 23. Figure 29 is a perspective view of the inner cylinder 23.

The inner cylinder 23 in the embodiment includes the outer tube 231, the fluid discharge tube 232, and the Y-shaped connector 2A. The fluid discharge tube 232 has the discharge hole 232a with the distal end (the leading end) 23a open, and communicates with the fluid discharge port 2Ab of the Y-shaped connector 2A. Furthermore, the aperture 25 communicates with the perfusion port 2Aa of the Y-shaped connector 2A through the space (the lumen) 26 formed between the inner surface of the outer tube 231 and the outer surface of the fluid discharge tube 232. Furthermore, the inner cylinder 23 has the inner cylinder operational bar 24, and as mentioned above, the operational bar 24 is removably bound to the middle cylinder 22 to become one-body with the middle cylinder 22, and then the one-body or only the inner cylinder 23 can control advancing or retracting with respect to the outer cylinder 21. In the embodiment, the inner cylinder operational bar 24 is installed on the leading end of the Y-shaped connector 2A and is an arm-shaped from the Y-shaped connector 2A toward the leading end side. Furthermore, the leading end of the inner cylinder operational bar 24 has a concave part 24a which can fit the convex part 223b of the operational bar 223 of the middle cylinder 22. Furthermore, the inner cylinder operational bar 24 of the inner cylinder 23 sequentially fits the notch structure 213c formed in the outer cylinder 21 to control advancing, and furthermore, the distal part (the leading end side) 2Ac of the Y-shaped connector 2A abuts against the proximal end (the rear end) 21b of the outer cylinder 21 to prevent the over-insertion of the inner cylinder 23.

Also in the embodiment, if required, other embodiments shown in Figures 14 to 16 can be adopted.

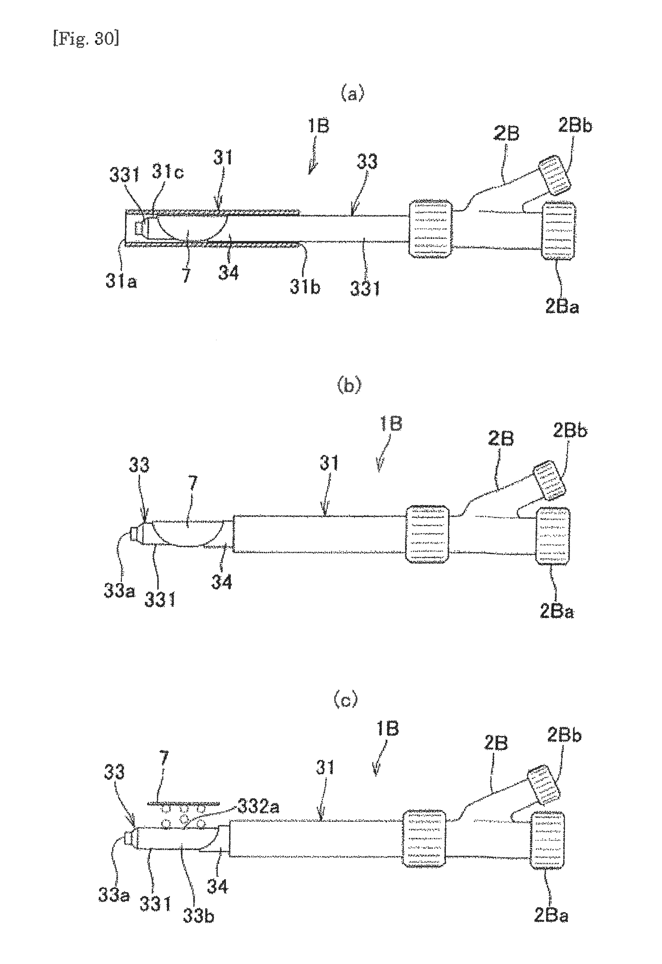

Figure 30 (a) is a schematic view of the injector for an eye 1B according to a third embodiment of the invention and shows a cross section of only the leading end of the outer cylinder 31. The injector for an eye 1B in the embodiment includes, as shown in Figure 30 (a), the outer cylinder 31, the inner cylinder 33 as the graft holding structure stored in the outer cylinder 31, and the Y-shaped connector (the hand proximal part) 2B. Furthermore, the leading end of the inner cylinder 33 has the graft holding part 33b, and the graft holding part 33b with holding the graft 7 is stored in the outer cylinder 31. Namely, the space (the inner space 31c) for storing the graft 7 is formed between the graft holding part 33b and the inner surface of the outer cylinder 31. Furthermore, the inner cylinder 33 has a fluid discharge hole 332a on the side face of the leading end (the graft holding part 33b) of the inner cylinder 33, and has the other discharge hole (the leading end aperture of the perfusion tube) for perfusate on the most leading end of the inner cylinder 33. The discharge hole 332a communicates with the fluid discharge port (the graft separating port) 2Bb of the Y shaped cannector 2B and the other discharge hole for perfusate communicates with the perfusion port 2Ba of the Y-shaped connector 2B. The fluid discharge port 2Bb is shown as a port, but in addition, may be a member with a plunger and a mechanism for directly pushing out a fluid.

Figure 30 (b) is a schematic side view in which the inner cylinder 33 of the injector for an eye 1B shown in Figure 30 (a) is advanced with respect to the outer cylinder 31 (the outer cylinder 31 is slid to the hand proximal side), the inner cylinder 33 is protruded from the leading end of the outer cylinder 31, and the graft 7 is exposed (taken out) from the outer cylinder 31. The injector for an eye 1B has a support notch 34 for holding and pushing out the graft 7. The support notch 34 has a step-shape protruded from the inner cylinder 33 which holds the graft 7, and works to prevent moving the graft 7 when the inner cylinder 33 holding the graft 7 is advanced with respect to the outer cylinder 31 at the time of inserting the graft 7 into an eye.

Figure 30 (c) is a schematic side view of the injector for an eye 1B in the state that the fluid is discharged from the fluid discharge hole 332a and the graft 7 is lifted by the fluid . For example, air, an intraocular infusion, and other liquid can be used as the fluid.

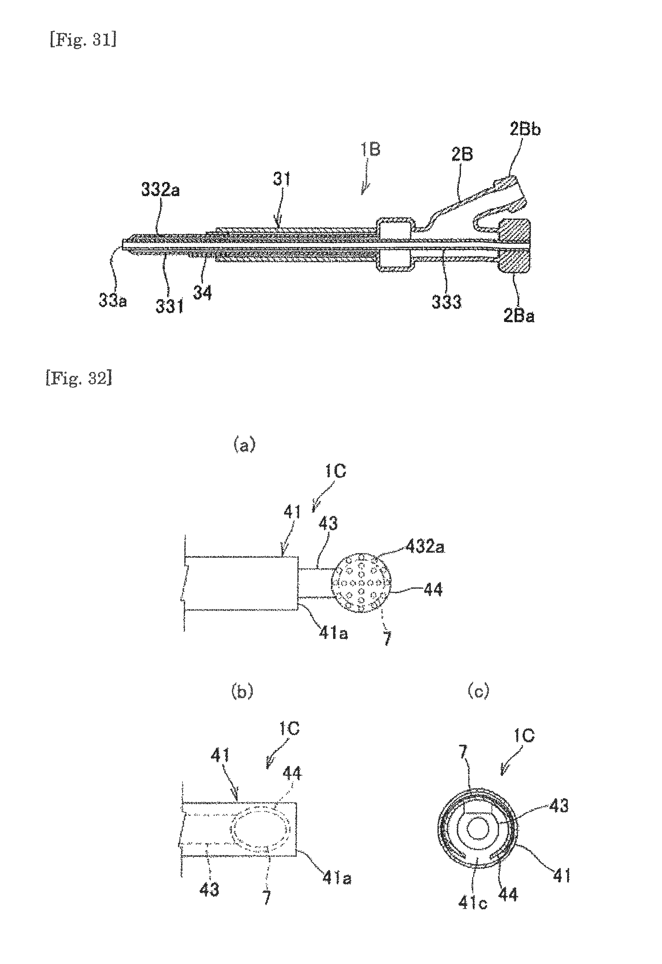

Figure 31 is a sectional view of the injector for an eye 1B shown in Figure 30 (b) (the graft 7 is not shown in Figure). The outer cylinder 31 has a tubular structure with both ends open. The inner cylinder 33 includes the outer tube 331, the perfusion tube 333 arranged in the outer tube 331, and the Y-shaped connector 2B arranged on the rear end side of the outer tube 331 and the perfusion tube 333. The fluid discharge hole 332a communicates with the fluid discharge port 2Bb through the space formed between the inner surface of the outer tube 331 and the outer surface of the perfusion tube 333, and the other discharge hole for perfusate communicates with the perfusion port 2Ba. Not shown in Figure 31, an O-ring and the like can be equipped inside of the outer cylinder 31 for controlling clearance with the inner cylinder 33, and can prevent backflow and properly control the resistance when sliding.

Figure 32 shows an outline of the leading end of the injector for an eye 1C according to a fourth embodiment of the invention. Figure 32 (a) is a schematic view showing the state in which the graft holding structure holding the graft 7 protrudes from the distal end (the leading end) 41a of the outer cylinder 41 of the injector for an eye 1C and an expander 44 is expanded. Figure 32 (b) is a schematic view showing the state in which the graft holding structure is stored inside the outer cylinder 41, and Figure 32 (c) is a schematic view showing the elevation view of Figure 32 (b).

In the embodiment, especially the graft holding structure includes a cylinder-shaped structure 43 and the expander 44. The expander 44 is installed on the leading end of the cylinder-shaped structure 43 and the expander 44 is formed so that a fluid could be fed through an inner space part of the cylinder-shaped structure 43. Furthermore, the expander 44 includes the graft holding part 43b and the graft holding part 43b can hold the graft 7 (in Figure, shown by two-dot chain lines). Furthermore, the expander 44 is movable by the fluid pressure of a fed fluid, when contracted, the expander 44 is folded so as to be stored in an inner space 41c of the outer cylinder 41 with the graft 7 becoming an outer surface (see Figure 32 (c)), and when expanded, the expander 44 can be expanded such as wing stretching to a flat shape (see Figure 32 (a)).

The cylinder-shaped structure 43 or the expander 44 may have the fluid discharge holes 432a for flowing a fluid toward the inner surface of the graft 7, and in the embodiment, the expander 44 having the discharge holes 432a on the surface of the expander 44 is illustrated. The discharge holes 432a do not communicate with the inside of the expander 44 and is separately formed.

### Example 1

The injector for an eye according to the present invention (hereinafter, sometimes simply referred to as the injector) will be described in detail with reference to the following Examples, but the invention is not limited to the Examples.

### [Example 1]

### <Manufacture of the graft containing the corneal endothelial cells and the Descemet's membrane>

Fresh piglet eyeballs were purchased from a public corporation for meat-packing and the inner layers of the corneal were carefully peeled. Then, the deep lamellar was punched using a trepan (a diameter of 8 mm) to prepare the sheet-shaped graft (the endothelial cell layer, the Descemet's membrane, and about 1/4 of the corneal stroma).

### <A graft insertion test by the injector for an eye #1>

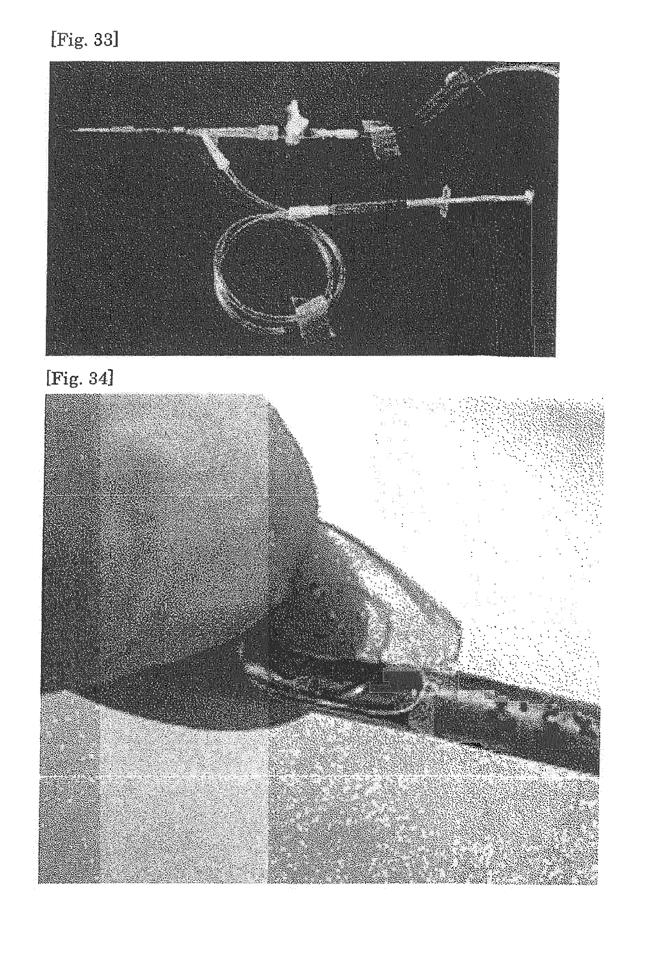

By using the injector for an eye #1 with a surface coating of polyethylene glycols shown in Figure 33 (slidability: a rubber packing), the prepared graft was inserted into a piglet eyeball to deliver to backside of the cornea. In the following transplant Examples, the piglet eyeballs whose intraocular pressures were equally kept even when the eyeball walls were pressured with tweezers were used.

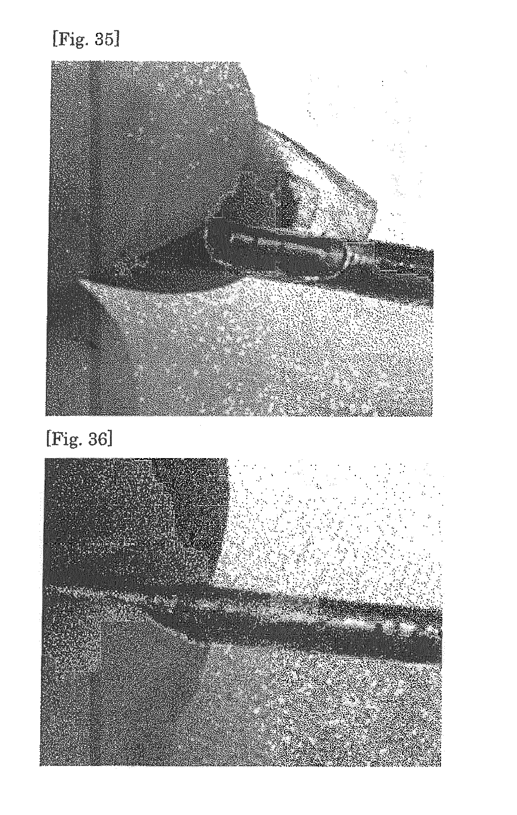

A small incision with a size of about 5 mm was made about 0.5 mm away from the corneal margin of the piglet eyeball to communicate with the anterior chamber. The graft was placed on the hinge structure of the outer cylinder (Figure 34), the graft holding part of the graft holding structure having the middle cylinder and the inner cylinder was slid on the graft (Figure 35), the corneal endothelial side of the graft was coated with a drop of hyaluronic acid for protecting the graft, and the gate was closed (Figure 36). After checking the hinge structure pinching, the hinge structure was filled with an intraocular infusion, and the graft holding structure was slid to slide the graft to the leading end of the outer cylinder.

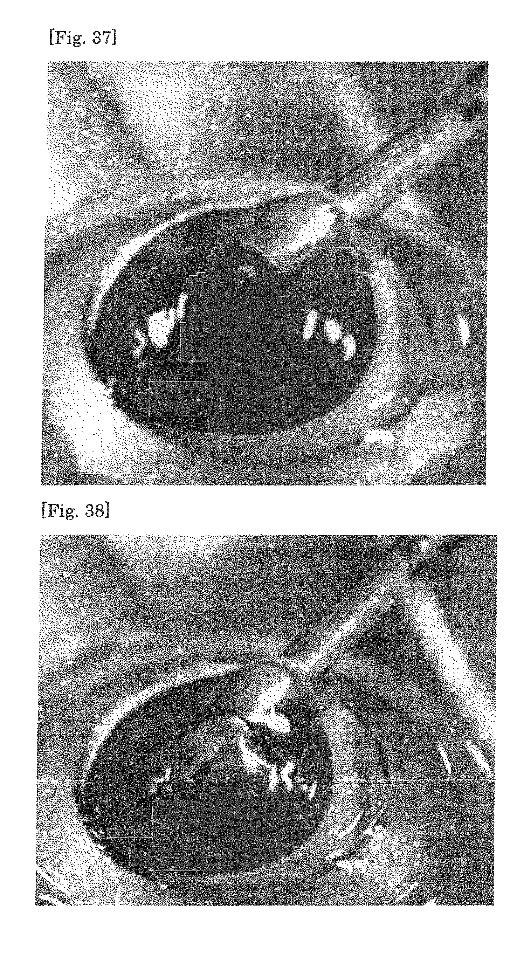

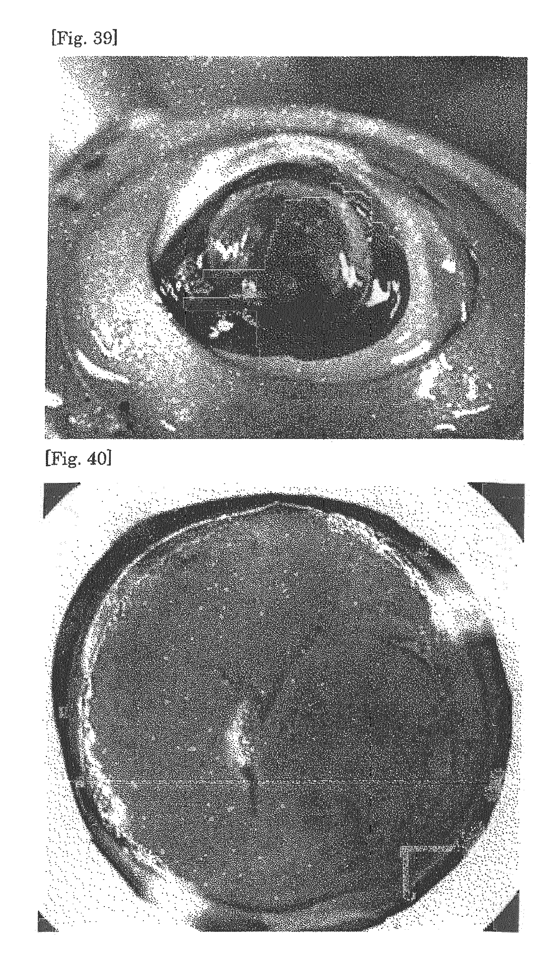

The injector for an eye was pointed so that, with respect to the insert direction, the major axis of the graft (when the graft is placed on the graft holding part, the uppermost axis on the graft with respect to the gravity direction, hereinafter the same applies) would be up and the edge (when the graft is placed on the graft holding part, the lowest edge with respect to the gravity direction, hereinafter the same applies) would be down. With an intraocular infusion (BSS PLUS manufactured by Alcon) slowly discharging from the perfusion lumen, the leading end of the injector was slowly inserted about 3 mm through the small incision (Figure 37), and when the intraocular pressure became high (when the anterior chamber was formed), the intraocular infusion was stopped. Next, the graft holding structure (the middle cylinder and the inner cylinder) was slid 4.5 mm with respect to the outer cylinder (the Y-shaped connector of the inner cylinder hit the side face of the proximal end of the outer cylinder to prevent the over-insertion of the inner cylinder), furthermore only the middle cylinder was slid 4.5 mm (simultaneously, the over-insertion of the middle cylinder was prevented by the lock mechanism installed in the outer cylinder) to expose the whole graft into the anterior chamber (Figure 38). Then, 0.5 mL of clean air was discharged from the discharge hole to deliver the graft to the transplant site. After the graft was stuck to the backside of the cornea, only the inner cylinder was slowly pulled by 3 cm toward the outside of the eye (a communicating way was formed) to relieve the intraocular pressure, and then the injector was slowly removed (Figure 39).

After completion of the graft delivery, the cornea was carefully incised, the inserted graft was taken out to examine the cell damage to the corneal endothelial cells caused by the injector by using the trypan blue-alizarin red staining. As a result, the graft inserted by using the injector had no damage (Figure 40). Accordingly, it was shown that, by using the injector, the graft can be inserted into an eye without any damage to the cells and delivered to the intended transplant site with keeping the anterior chamber (Table 1).

### [Example 2]

### <A graft insertion test by the injector for an eye #2>

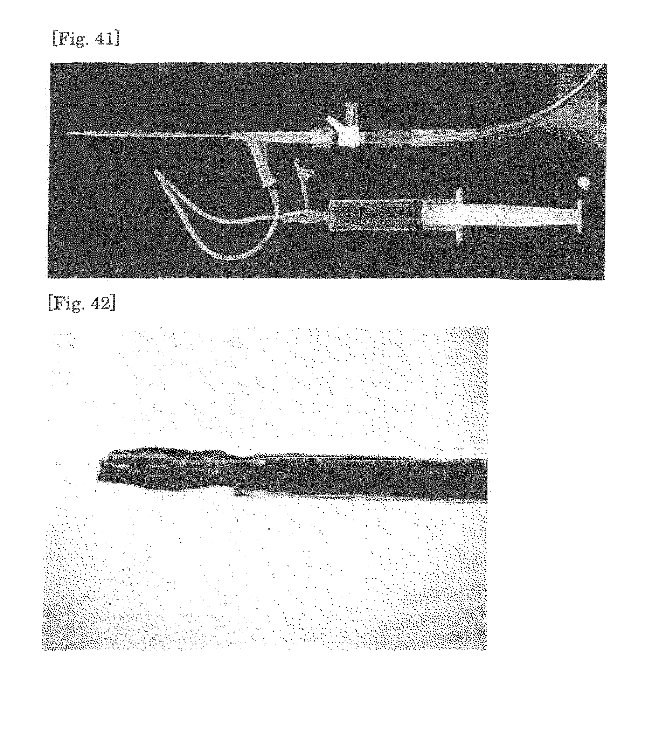

By using the injector for an eye #2 with a surface lubricious coating of polyethylene glycols shown in Figure 41 (slidability: a rubber packing), the prepared graft was inserted into the piglet eyeball to deliver to the backside of the cornea.

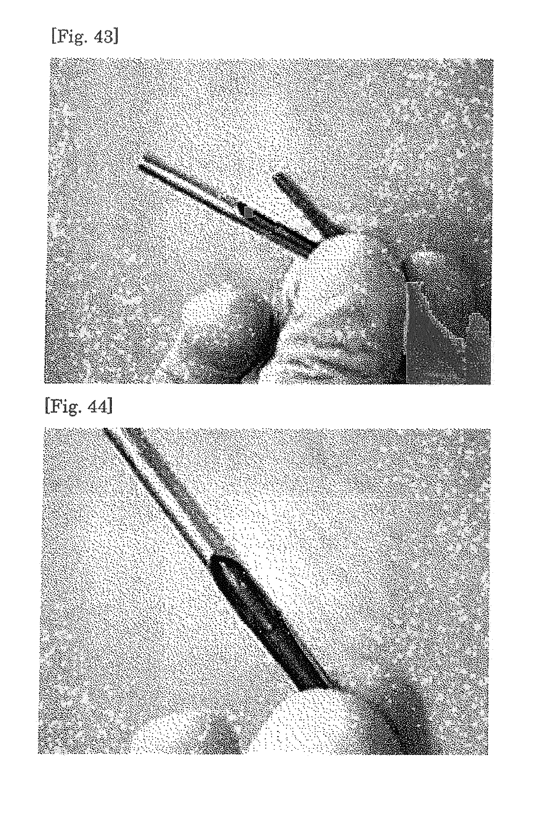

In the same manner as in Example 1, a small incision with a size of about 5 mm was made about 1 mm away from the corneal margin of the piglet eyeball to communicate with the anterior chamber. A drop of hyaluronic acid and a graft was placed on the graft holding part of the graft holding structure having the middle cylinder and the inner cylinder (Figure 42), and the graft holding structure was slowly installed in the outer cylinder having a trumpet-shaped inner structure (Figures 43 and 44). Then, the graft holding structure was slid to slide the graft to the leading end of the outer cylinder.

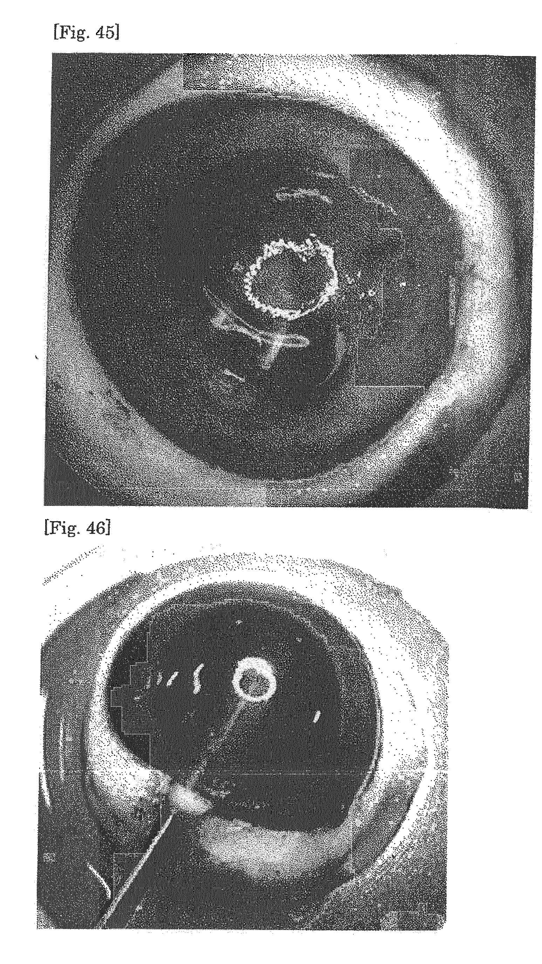

The injector was pointed so that the major axis of the graft would be up with respect to the insert direction and the edge would be down. With an intraocular infusion slowly discharging from the perfusion lumen, the injector was slowly inserted about 3 mm through the small incision, and when the intraocular pressure became high (when the anterior chamber was formed), the intraocular infusion was stopped. Next, the graft holding structure (the middle cylinder and the inner cylinder) was slid 4.5 mm form the outer cylinder (the Y-shaped connector of the inner cylinder hit the side face of the proximal end of the outer cylinder to prevent the over-insertion of the inner cylinder), furthermore only the middle cylinder was slid 4.5 mm (simultaneously the over-insertion of the middle cylinder was prevented by the lock mechanism formed in the outer cylinder) to expose the whole graft into the anterior chamber. Then, 0.3 mL of clean air was discharged from the discharge hole to deliver the graft to the transplant site. After the graft was stuck, only the inner cylinder was slowly slid to relieve the intraocular pressure, and then the injector was slowly removed (Figure 45).

After completion of the graft delivery, in the same manner as in Example 1, the cell damage to the corneal endothelial cells caused by the injector was examined by using the trypan blue-alizarin red staining. As a result, the graft inserted by using the injector had no damage (not shown in Figure). Accordingly, it was shown that, by using the injector, the graft can be inserted into an eye without any damage to the cells and delivered to the intended transplant site with keeping the anterior chamber (Table 1).

### [Example 3]

### <A graft insertion test by the injector for an eye #3>

The insertion test was performed in the same manner as in Examples 1 and 2 except that the injector #3 was used. The injector #3 was the injector #1 having a surface without lubricious coating and its slidability was controlled by only each cylinder diameter (compliance). The obtained result was similar to those of Examples 1 and 2 (Table 1).

It was shown that the object can be achieved by even an untreated surface with lubricious coating and slide control with the cylinder diameter.

### [Example 4]

### <A graft insertion test of the injector for an eye #1 in a different usage pattern>

In the same manner as in Example 1, the graft was slid to the leading end of the outer cylinder (the Y-shaped connector of the inner cylinder hit the side face of the proximal end of the outer cylinder to prevent the over-insertion of the inner cylinder). With an intraocular infusion slowly discharging from the perfusion lumen, the injector was slowly inserted about 3 mm through the small incision, and when the intraocular pressure became high (when the anterior chamber was formed), the intraocular infusion was stopped. Next, only the middle cylinder was slid 9 mm with respect to the outer cylinder and the inner cylinder (simultaneously, the over-insertion of the middle cylinder was prevented by the lock mechanism formed in the outer cylinder) to expose the whole graft into the anterior chamber. Then, after the leading end of the injector was slowly rippled to separate and expand the graft, the injector was removed from the eye.

After completion of the graft delivery, in the same manner as in Examples 1 to 3, the cell damage to the corneal endothelial cells caused by the usage pattern was examined by using the trypan blue-alizarin red staining. As a result, the graft inserted by using the injector had no damage (not shown in Figure). Accordingly, it was shown that, also in the usage pattern, the graft can be inserted into an eye without any damage to the cells.

### [Example 5]

### <A graft insertion test by the injector for an eye #4>

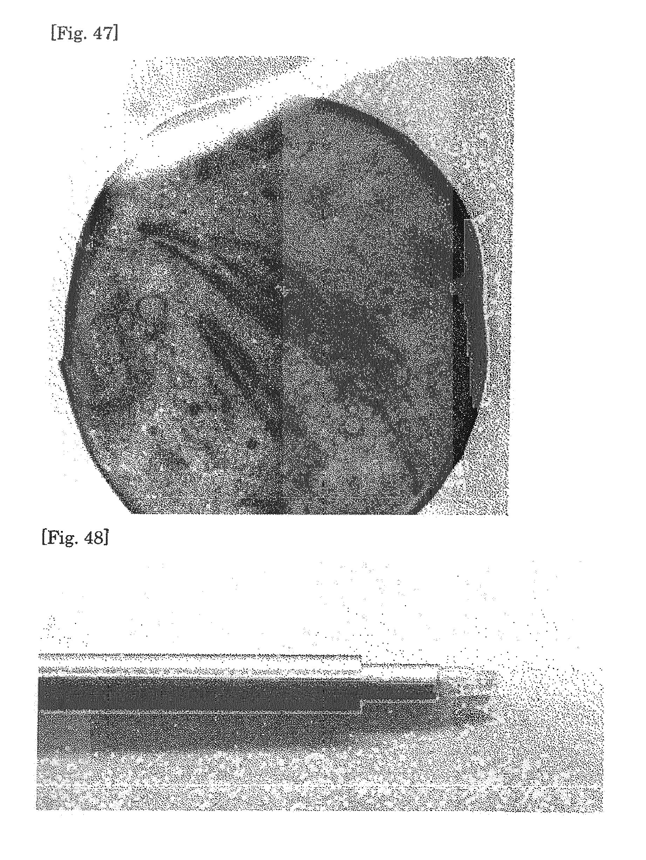

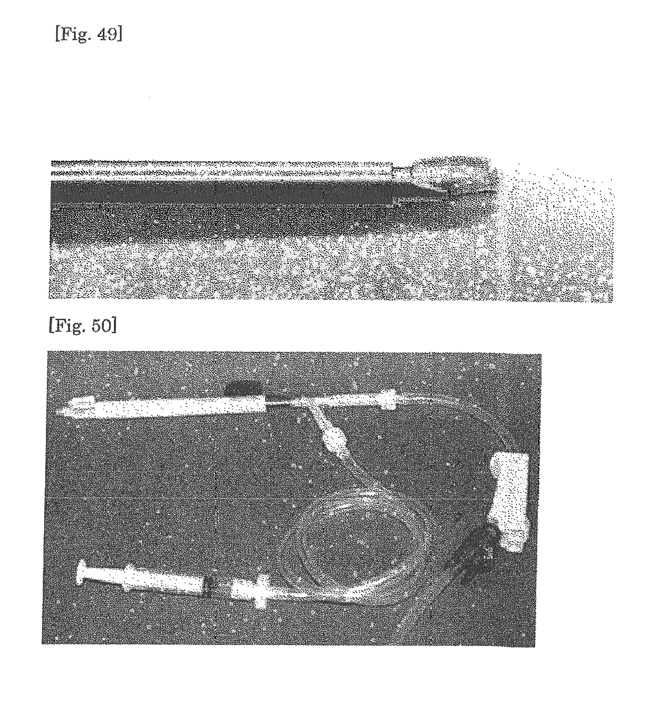

By using the injector for an eye #4 with the graft holding structure shown in Figures 48 and 49, the prepared graft derived from the rabbit cornea was inserted into a rabbit eyeball to deliver to the backside of the cornea. In the following transplant Examples, the rabbit eyeballs whose intraocular pressures were equally kept even when the eyeball walls were pressured with tweezers were used.

A small incision with a size of about 5 mm was made about 1 mm away from the corneal margin of the rabbit eyeball to communicate with the anterior chamber. The graft was placed on the graft holding structure (Figure 49) to store in the outer cylinder, the injector was slowly inserted through the incision, the intraocular infusion was flowed from the perfusion lumen in order to keep the intraocular pressure (the bottle containing the intraocular infusion connected to the perfusion lumen was placed at a height of 30 cm above the cornea). Next, the graft holding structure was slid form the outer cylinder, and when the graft came under the cornea, the intraocular infusion was discharged from the fluid discharge hole to deliver the graft to the transplant site. At this time, the eyeball wall was pressured with tweezers in order to examine an intraocular pressure, and it was shown that an enough tension was held.

After completion of the graft delivery, the cornea was carefully incised, and the inserted graft was taken out to examine cell damage to the corneal endothelial cells caused by the injector by a fluorescent microscope observation (Leica DM-IRB) using the Live/Dead assay (Molecular Probes) (not shown in Figure). As a result, the whole area of the endothelial cells of the graft inserted by using the injector was positive in the Live staining and most of the endothelial cells were negative in the Dead staining). Accordingly, it was shown that, by using the injector, the graft can be inserted into an eye without any damage to the cells and delivered to the transplant site (Table 1).

### [Example 6]

### <A graft insertion test by the injector for an eye #5>

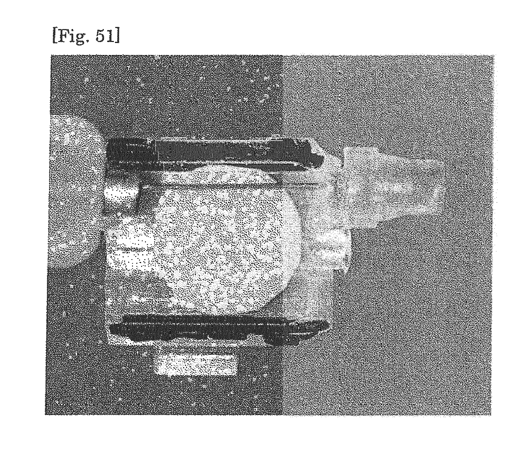

By using the injector for an eye #5 having the gate with the hinge structure at the aperture of the outer cylinder shown in Figures 50 and 51, the insertion test was performed in the same manner as in Examples 1 and 2. The obtained result was similar to those of Examples 1 and 2 (Table 1).

### [Comparative Example 1]

### <An insertion test by using tweezers for ophthalmology>

In the same manner as in Examples 1 to 3, the graft was inserted into a piglet eyeball by using tweezers for ophthalmology coated with hyaluronic acid (Figure 46) to examine cell damage (performed three times).

As a result, in all the cases, a part of the endothelial cells of the graft inserted by using the tweezers showed positive in the trypan blue-alizarin red staining, and especially the region of the endothelial cells gripped with the tweezers was strongly damaged (Figure 47). When the tweezers was removed from the eye and the graft was oriented with a cannula, an intraocular fluid flowed out and the anterior chamber was deflated.

### [Comparative Example 2]

In the same manner as in Example 5, the graft derived from the rabbit cornea was inserted into a rabbit eyeball by using tweezers for ophthalmology coated with hyaluronic acid to examine cell damage. As a result, it was shown that many of the endothelial cells of the graft inserted by using the tweezers showed positive in the Live staining, but a part of the endothelium at the region gripped with the tweezers was peeled off (not shown in Figure). Furthermore, when the tweezers was removed from the eye, the intraocular fluid flowed out and the eyeball (the anterior chamber) was deflated. The concluded results are shown in Table 1.

In Table, "⊚" means that the anterior chamber was well kept when the graft insertion was completed and the equipment was removed, "○" means that the anterior chamber becomes shallow when comparing with "⊚", and "Δ" means that the anterior chamber was deflated.

**[Table 1]**

| | Formation of the anterior chamber | Staining for Cell damage |
|---|---|---|
| Example1 | ⊚ | Negative |
| Example2 | ⊚ | Negative |
| Example3 | ⊚ | Negative |
| Example4 | ○ | Negative |
| Example5 | ⊚ | Negative* |
| Example6 | ⊚ | Negative |
| Comparative Example 1 | Δ | the region gripped with tweezers: positive |
| Comparative Example 2 | Δ | the region gripped with tweezers: positive* |

| | | |
|---|---|---|
| *Live/Dead Assay | | |

## Claims

1. An injector for an eye for inserting a graft into an eyeball, comprising: an outer cylinder and
a graft holding structure arranged inside the outer cylinder to be slidable with respect to the outer cylinder,
the graft holding structure includes a graft holding part capable of being stored inside the outer cylinder, the graft holding part forming a space capable of storing a graft between an inner surface of the outer cylinder and the graft holding part at the time of the graft holding part being stored in the outer cylinder, and
the graft holding part protrudes from the outer cylinder at the time of the graft holding structure being advanced and the graft is able to be taken out from the outer cylinder, and
is provided with a discharge hole for an intraocular infusion for keeping intraocular pressure or forming anterior chamber.

2. The injector for an eye according to Claim 1, wherein the graft holding structure includes a middle cylinder and an inner cylinder arranged in the middle cylinder to be slidable with respect to the middle cylinder, and
the middle cylinder is configured to push out the graft of the graft holding part by sliding of the middle cylinder with respect to the outer cylinder.

3. The injector for an eye according to Claim 1, wherein the graft holding structure includes a middle cylinder and an inner cylinder arranged in the middle cylinder to be slidable with respect to the middle cylinder, and
the middle cylinder is configured to push out the graft of the graft holding part by sliding of the middle cylinder with respect to the inner cylinder.

4. The injector for an eye according to Claim 1, wherein the graft holding structure includes a middle cylinder and an inner cylinder arranged in the middle cylinder to be slidable with respect to the middle cylinder, and
the middle cylinder is configured to push out the graft of the graft holding part by sliding of the middle cylinder with respect to the outer cylinder and sliding of the middle cylinder with respect to the inner cylinder.

5. The injector for an eye according to anyone of Claims 1 to 4, wherein the discharge hole is formed on the graft holding structure.

6. The injector for an eye according to Claim 5, wherein the discharge hole is formed on the inner cylinder, or on the middle cylinder.

7. The injector for an eye according to anyone of Claims 1 to 6, wherein an aperture at a leading end of the outer cylinder communicates with an aperture formed on a side face or a rear end part of the outer cylinder by sliding the graft holding structure toward the outside of the eye.

8. The injector for an eye according to anyone of Claims 2 to 4 and 6, wherein an aperture at a leading end of the outer cylinder or the middle cylinder communicates with the aperture formed on the side face or the rear end part of the outer cylinder by sliding the middle cylinder or the inner cylinder constituting the graft holding structure toward the outside of the eye.

9. The injector for an eye according to Claim 8, wherein pressure outlets formed on the middle cylinder and the outer cylinder communicate with an inner space of the middle cylinder formed by sliding the inner cylinder toward the outside of the eye, and thereby the aperture at the leading end of the outer cylinder communicates with the pressure outlet formed on the outer cylinder.

10. The injector for an eye according to anyone of Claims 1 to 9, wherein an inner surface of the outer cylinder has a lubricious coating.

11. The injector for an eye according to anyone of Claims 1 to 9, wherein an outer surface of the graft holding structure has a lubricious coating.

12. The injector for an eye according to anyone of Claims 1 to 9, wherein an inner surface of the outer cylinder and an outer surface of the graft holding structure have a lubricious coating.

13. The injector for an eye according to anyone of Claims 2 to 4, 6, 8 and 9, wherein an inner surface of the outer cylinder, an outer surface of the inner cylinder, and an outer surface of the middle cylinder have a lubricious coating.

14. The injector for an eye according to anyone of Claims 2 to 4, 6, 8 and 9, wherein an inner surface of the outer cylinder and an outer surface of the middle cylinder have a lubricious coating.

15. The injector for an eye according to anyone of Claims 2 to 4, 6, 8 and 9, wherein an inner surface of the outer cylinder and an outer surface of the inner cylinder and the middle cylinder have a lubricious coating.

16. The injector for an eye according to anyone of Claims 1 to 15, further comprising an aperture for installing the graft, wherein the aperture is on the side face of the outer cylinder and the side face is located where the graft holding part is stored when the graft holding structure is arranged in the outer cylinder.

17. The injector for an eye according to Claim 16, further comprising an opening and closing gate for sealing the aperture.

18. The injector for an eye according to anyone of Claims 1 to 17, wherein the graft holding structure includes a graft holding part, the graft holding part forming a space capable of storing a graft between an inner surface of the outer cylinder and the graft holding part at the time of the graft holding part being stored in the outer cylinder, said graft including at least one selected from corneal endothelial cell, Descemet's membrane of cornea, corneal stroma, iris tissue, capsula lentis, retina, or a cultured composition.

19. The injector for an eye according to Claim 18, wherein the graft holding structure includes a graft holding part, the graft holding part forming a space capable of storing a graft between an inner surface of the outer cylinder and the graft holding part at the time of the graft holding part being stored in the outer cylinder, the graft including a sheet piece used for transplantation of corneal endothelial cells.
